# EUROPEAN PATENT APPLICATION

(11) **EP 3 660 039 A1**
(43) Date of publication of application: **03.06.2020**
(21) Application number: 18209671.9
(22) Date of filing: 30.11.2018
(51) Int. Cl.: C07K 14/55, C07K 16/18, A61K 47/68, A61P 35/00

(54) **IL2 IMMUNOCONJUGATES**

(71) Applicant: Philogen S.p.A., 53100 Siena (IT)
(72) Inventor: VILLA, Alessandra, CH-8112 Otelfingen (CH); ONGARO, Tiziano, CH-8112 Otelfingen (CH)
(74) Representative: Mewburn Ellis LLP

(57) **Abstract**

The present application relates to a conjugate for targeting an agent, such as a therapeutic or diagnostic agent, to tissues *in vivo.* In particular, it relates to conjugates for targeting the extracellular matrix (ECM) of tissues, particularly tumour neovasculature, and to therapeutic use of such conjugates in the treatment of a disease/disorder, such as cancer. In particular the application relates to immunocytokines for targeting interleukin-2 (IL2) to ECM components associated with neoplastic growth and/or angiogenesis.

## Description

### Field

The present invention relates to a conjugate for targeting an agent, such as a therapeutic or diagnostic agent, to tissues *in vivo.* In particular, it relates to conjugates for targeting the extracellular matrix (ECM) of tissues, particularly tumour neovasculature, and to therapeutic use of such conjugates in the treatment of a disease/disorder, such as cancer. In particular the invention relates to immunocytokines for targeting interleukin-2 (IL2) to ECM components associated with neoplastic growth and/or angiogenesis.

### Background

Cytokines are key mediators of innate and adaptive immunity. Many cytokines have been used for therapeutic purposes in patients with advanced cancer, but their administration is typically associated with severe toxicity, hampering dose escalation to therapeutically active regimens and their development as anticancer drugs. To overcome these problems, the use of 'immunocytokines' (i.e. cytokines fused to antibodies or antibody fragments) has been proposed, with the aim to concentrate the immune-system stimulating activity at the site of disease while sparing normal tissues (Savage *et al.,* 1993; Schrama *et al.,* 2006; Neri & Bicknell, 2005; Dela Cruz *et al.,* 2004; Reisfeld *et al.,* 1997). However, genetically fusing a cytokine to an antibody or to an antibody fragment creating an "immunocytokine", does not always result in an immunocytokine that retains the ability to target the tumor of the antibody. For example, in certain Interleukin-7 fusions (Pasche et al. (2011) J Biotechnology, 154, 84-92) the tumor targeting was completely abrogated, while in certain GM-CSF fusions (Kaspar et al. (2007) Cancer Res, 67, 4940-4948) the tumor targeting ability was found to be dose dependent.

IL2 is a four α helix bundle cytokine produced by T helper 1 cells and plays an essential role in the activation phases of both specific and natural immune responses. IL2 promotes proliferation and differentiation of activated T and B lymphocytes and of natural killer (NK) cells and induces cytotoxic T cell (CTL) activity and NK/lymphokine-activated killer (LAK) cell antitumor cytotoxicity. IL2 has been approved for the treatment of several human cancers. Administration of recombinant IL2 (rIL2) alone or in combination with adoptively transferred lymphoid cells has been shown to result in the regression of established tumors in both animal models and patients. However, the *in vivo* therapeutic efficacy of IL2 is limited by its rapid clearance and, at high doses severe toxicity mainly related to a vascular leak syndrome.

To overcome the drawbacks associated with IL2 therapy, delivery of IL2 to the tumor site by means of an antibody directed against tumor-associated marker to increase local concentrations of IL2 at the tumour site, as well as reduce toxicities associated with systemic administration of IL2 has been proposed. In particular, the concentration of cytokines at the level of tumour blood vessels is an attractive therapeutic strategy as the tumour neovasculature is more accessible to intravenously administered therapeutic agents than tumour cells, which helps avoid problems associated with the interstitial hypertension of solid tumours (Jain & Baxter, 1998). In addition, angiogenesis is characteristic of most aggressive solid tumours (Folkman, 1995). Angiogenesis describes the growth of new blood vessels from existing blood vessels. Tumours can induce angiogenesis through secretion of various growth factors (e.g. Vascular Endothelial Growth Factor). Tumour angiogenesis allows tumours to grow beyond a few millimetres in diameter and is also a prerequisite for tumour metastasis. New blood vessels formed as the result of angiogenesis form the neovasculature of the tumour or the tumour metastases. Targeting IL-2 to the neovasculature should allow the immunotherapy of a variety of different tumour types.

The alternatively spliced extra domains A (ED-A) and B (ED-B) of fibronectin and the A1 domain of tenascin-C represent three of the best-characterised markers of angiogenesis and have been reported to be expressed around the neo-vasculature and in the stroma of virtually all types of aggressive solid tumours. Furthermore, even non-solid cancers, such as leukaemia, may be amenable to treatment by targeting antigens of the neovasculature. WO2011/015333 described treating leukaemia, including acute myeloid leukaemia, by targeting the bone marrow neovasculature.

Three human monoclonal antibodies specific to these targets have been developed and moved to clinical trials: L19 (specific to ED-B; Pini *et al.,* 1998; WO1999/058570), F8 (specific to ED-A; Villa *et al.,* 2008; WO2008/120101) and F16 (specific to the A1 domain of tenascin-C; Brack *et al.,* 2006; WO2006/050834). In addition, immunocytokines based on L19, F8 or F16 are currently being investigated in Phase I, Phase II and Phase III clinical trials in patients with cancer and chronic inflammatory disease such as rheumatoid arthritis and endometriosis (Sauer *et al.,* 2009; Johannsen *et al.,* 2010). These immunocytokines include several immunocytokines comprising IL2.

L19-IL2 (darleukin) (WO2001/062298) has been tested in a variety of therapeutic regimens and combinations for treatment of different types of cancer (WO2007/115837, WO2009/089858, WO2013/010749, WO2013/045125, WO2018/115377, WO2018/154517) with good results. In this particular immunocytokine, the L19 antibody has always been used in the scFv configuration.

An F16-IL2 diabody conjugate (teleukin) is also being evaluated in clinical trials.
An F8-IL2 diabody conjugate has been shown to reduce tumour burden in mice (WO2008/120101 and WO2010/078945).

The immunocytokine format, as well as the format of the antibody fragment portion of the antibody, has been shown to have an impact on tumour targeting efficacy of the immunocytokine.

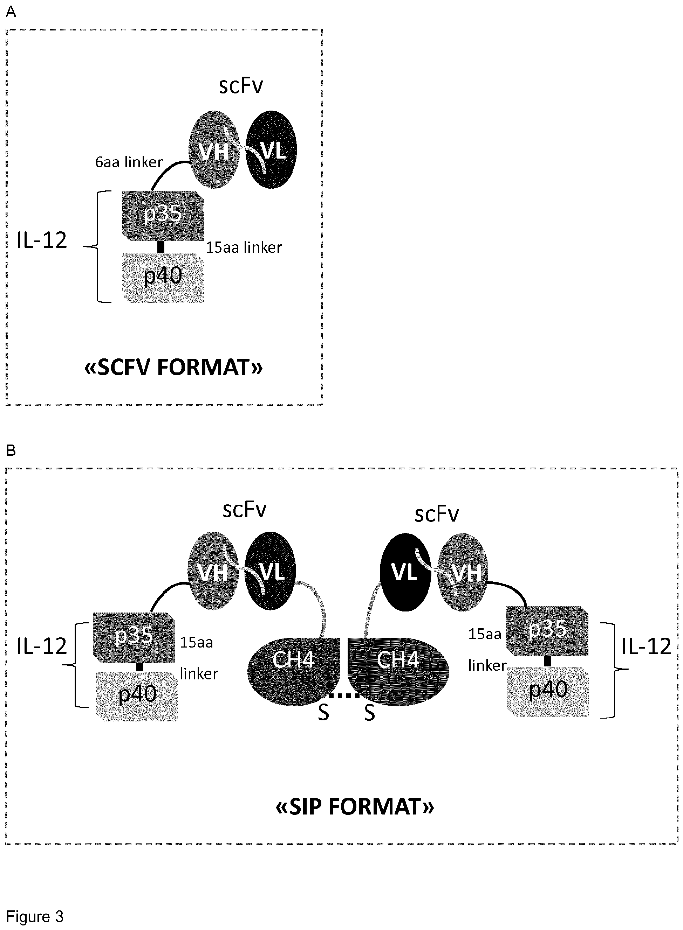

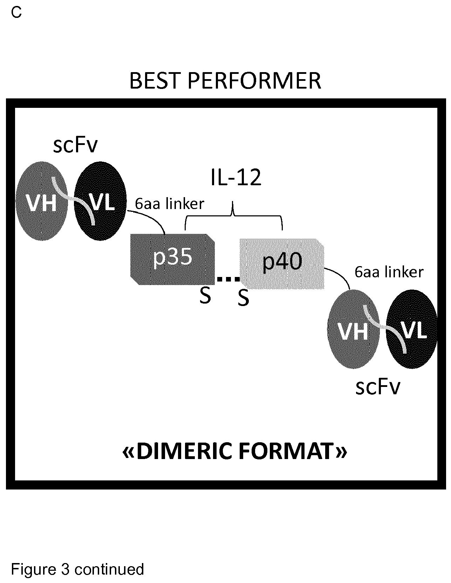

For example, in WO2006/119897 the L19 antibody was conjugates to Interleukin-12, a heterodimeric cytokine formed by the p35 and p40 subunits, in three different molecular formats schematically shown in Figure 3:
(i) two scFvs conjugated to either the p35 subunit or the p40 subunits held together by a disulphide bond ("dimeric format");
(ii) an SIP antibody conjugated to two IL12 molecules ("SIP format");
(iii) an scFv conjugated at its N-terminus to one IL12 molecule - a format previously disclosed in WO2001/062298 - ("scFv format").

The results of WO2006/119897 showed that the "dimeric format" had a superior tumor targeting ability as compared to the "SIP format" and "scFv format".

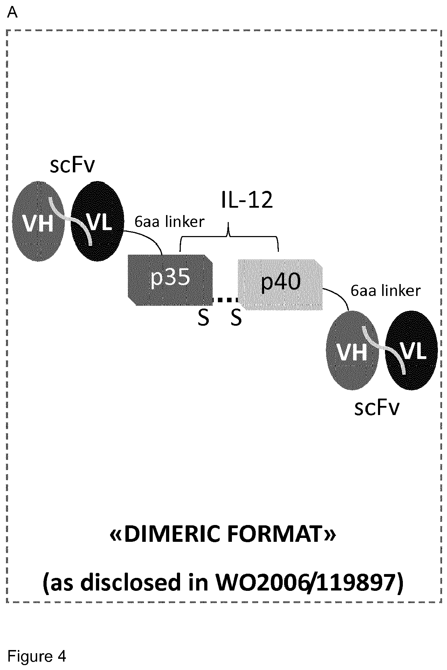

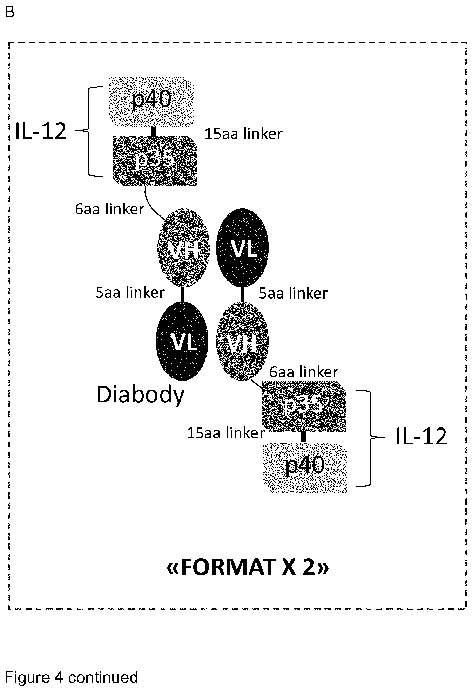

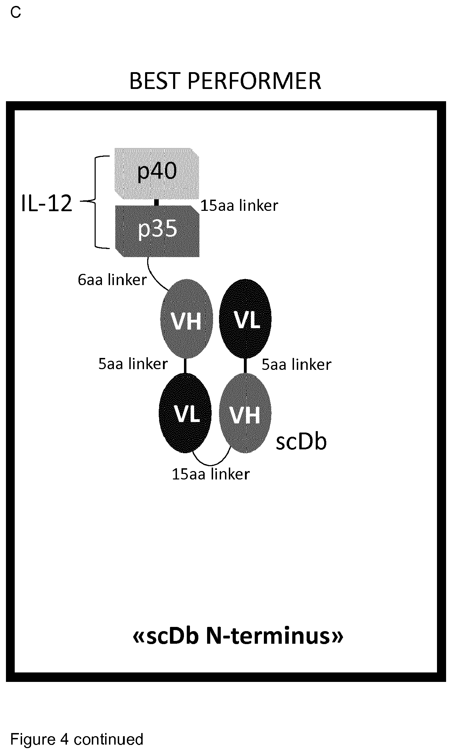

In WO2013/014149 the current applicant compared the tumor targeting ability of the "dimeric format" disclosed in WO2006/119897, with two new IL12 conjugates schematically shown in Figure 4:
(i) a single-chain diabody conjugated to one IL12 molecule at its N-terminus ("scDb N-terminus")
(ii) a diabody conjugated to two IL12 molecules at its N-terminus and at its-C-terminus "format X 2".

The results of WO2013/014149 showed that the "scDb N-terminus" conjugate had superior tumor targeting ability as compared to the "dimeric format" and "format X 2".

Furthermore, in WO2018/069467 the current applicant compared the tumor targeting ability of five different molecular formats in which different antibody fragments were conjugated to Interleukin-4 (IL4) a compact globular protein with many biological roles.

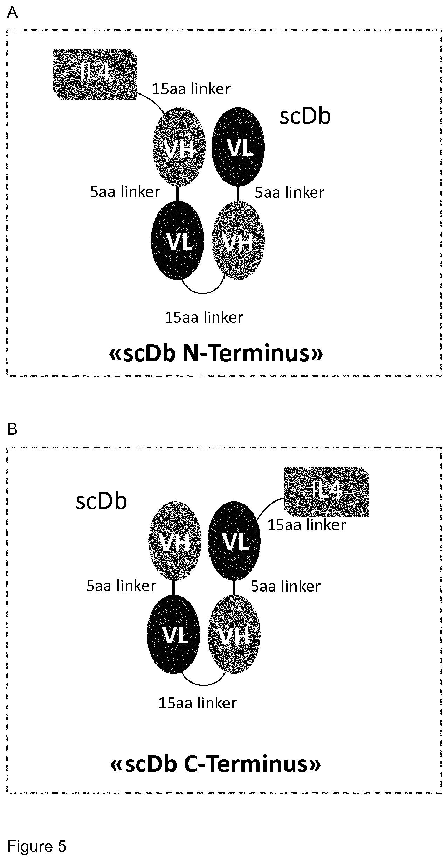

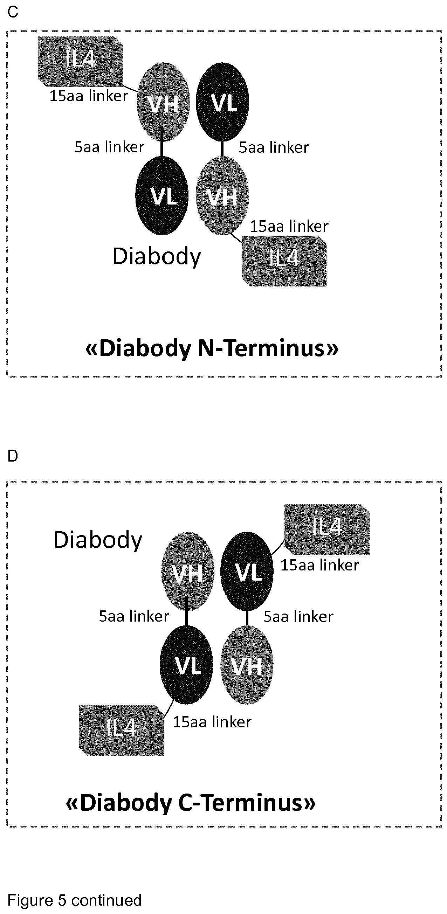

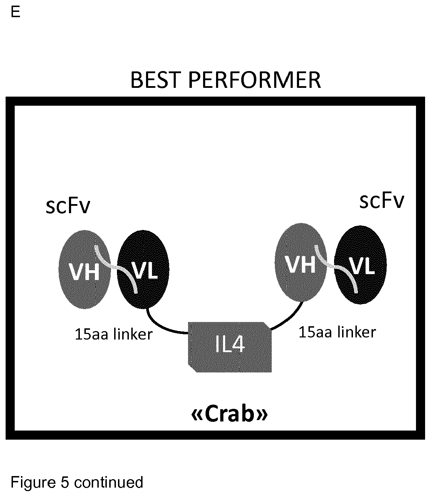

As schematically shown in Figure 5, the following conjugate formats were compared in WO2018/069467:
(i) a scDb with IL4 conjugated at its C-terminus ("scDb C-terminus")
(ii) a scDb with IL4 conjugated at its N-terminus ("scDb N-terminus")
(iii) a diabody with IL4 conjugated at its C-terminus ("Diabody C-terminus")
(iv) a diabody with IL4 conjugated at its N-terminus ("Diabody N-terminus")
(v) IL4 conjugated at its C-terminus and at its N-terminus to two scFv's ("Crab")

The results of WO2018/069467 showed that the "Crab" format had a superior tumor targeting ability as compared to the other four formats tested.

### Summary of the invention

The present invention relates to a conjugate comprising IL2 and a single-chain diabody. More specifically, the present invention relates to a conjugate comprising IL2 and a single-chain diabody, wherein the IL2 is linked to the C-terminus or the N-terminus of the single-chain diabody, preferably the C-terminus of the single-chain diabody.

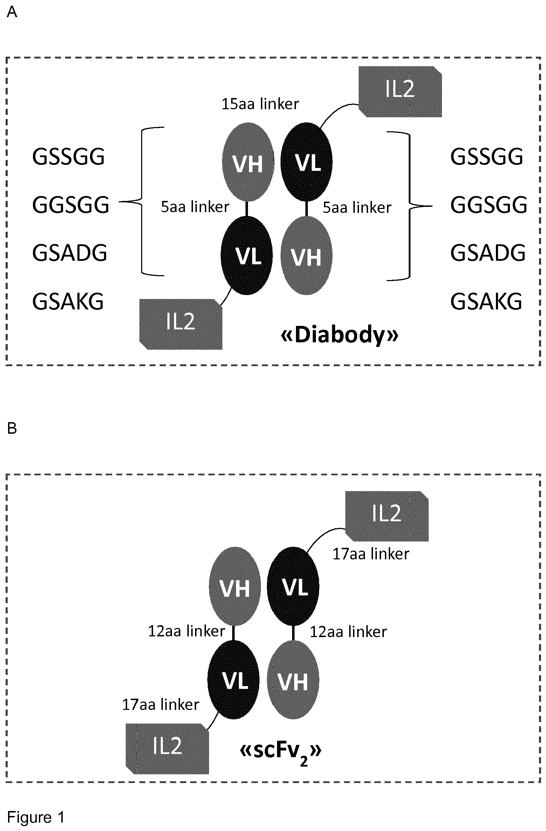

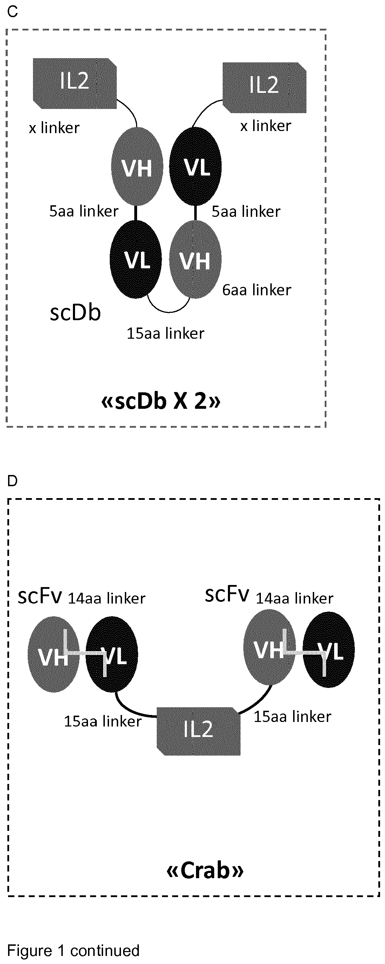

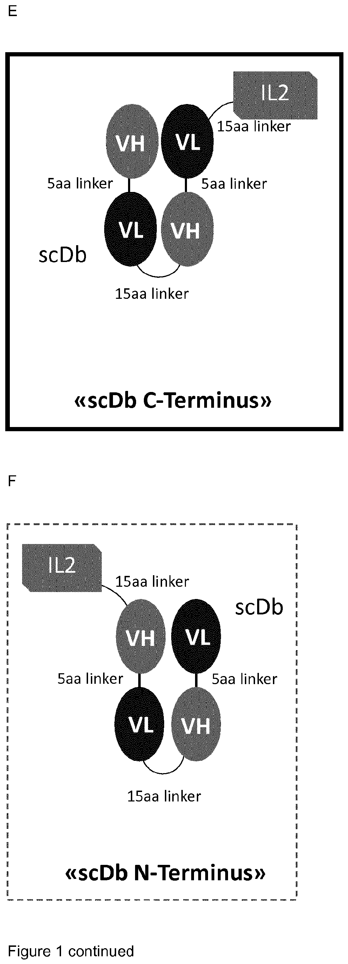

The invention is derived from work which compared the tumour-targeting properties of nine antibody-IL2 immunocytokines in six different formats. The formats tested are illustrated in **Figure 1****,** namely:
(A) a diabody comprising one of four different VH-VL domain linker sequences ("diabody");
(B) a single-chain variable fragment (scFv) with a 12 amino acid linker between VH and VL in which L19 scFv forms a dimer (scFv₂) as disclosed in WO2001/062298, Viti et al. (1999) and Borsi *et al.* (2002);
(C) a single-chain diabody (scDb) with IL2 conjugated at both its C-terminus and at its N-teminus ("scDb X 2");
(D) IL2 conjugated at its C-terminus and at its N-terminus to two scFv's ("Crab");
(E) a scDb with IL2 conjugated to its C-terminus ("scDb C-terminus"); and
(F) a scDb with IL2 conjugated to its N-terminus ("scDb N-terminus").

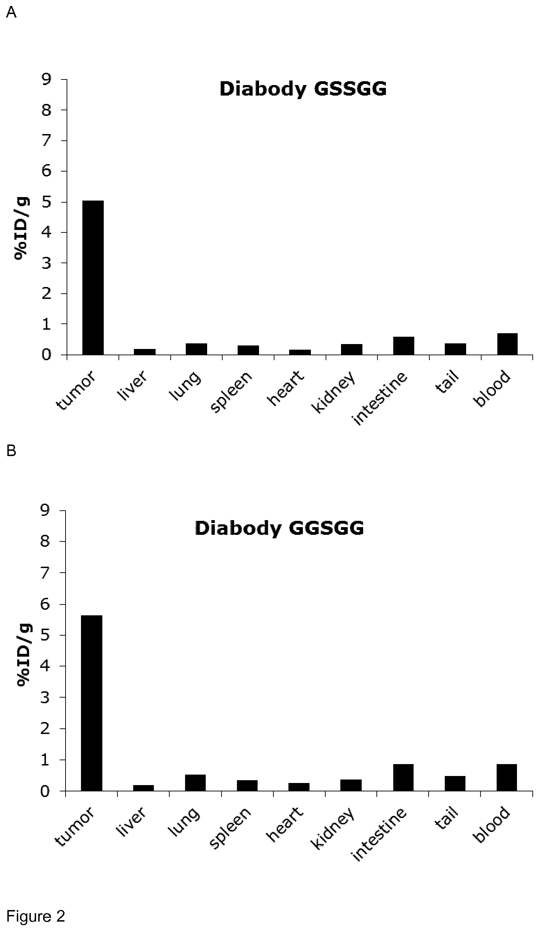

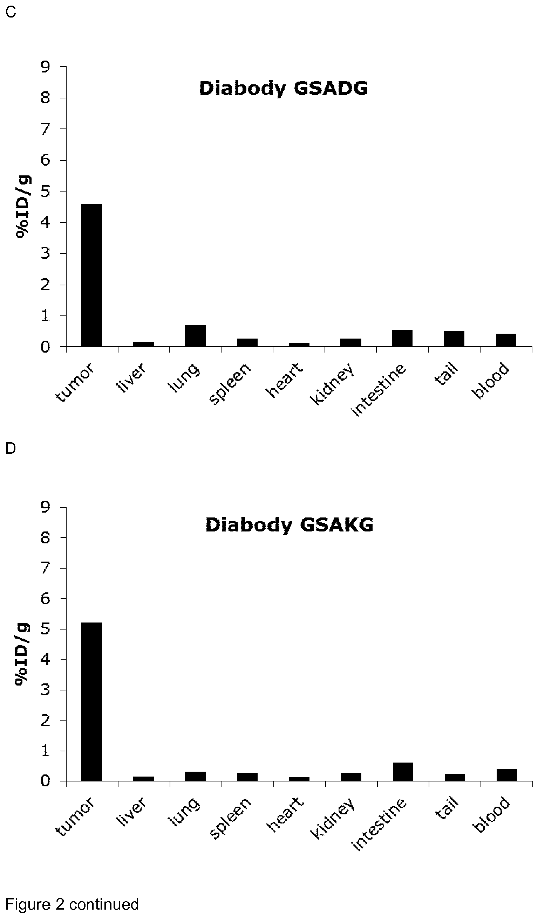

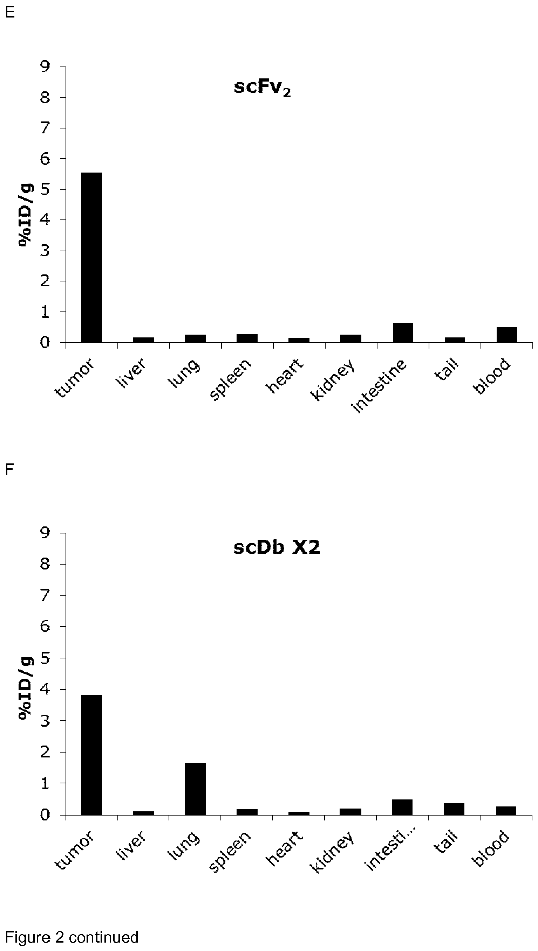

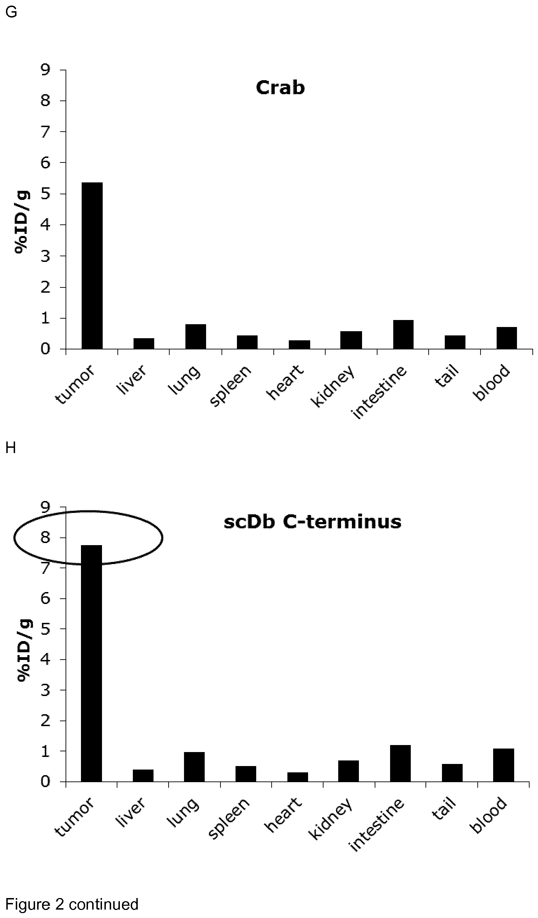

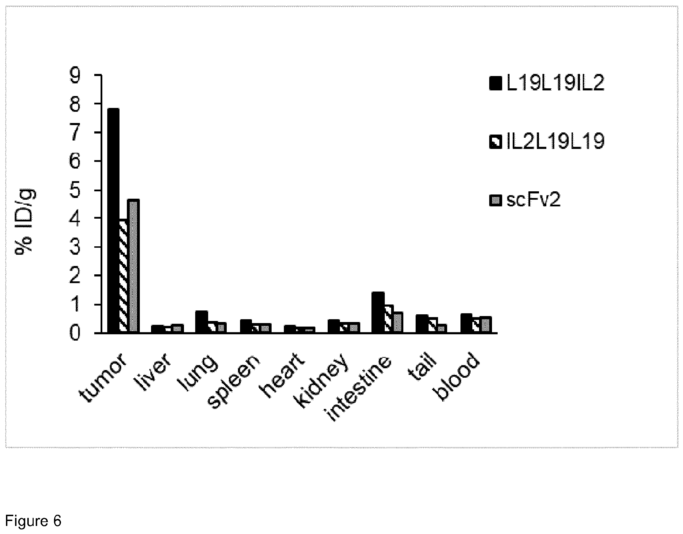

The single-chain diabody (scDb) comprising IL2 conjugated to the C-terminus of the single-chain diabody was surprisingly shown to have superior tumour targeting properties compared with all of the other immunocytokine formats tested. Specifically, 24 hours after injection into tumour-bearing mice, the "scDb C-terminus" conjugate reached a percentage injected dose/gram of tissue (%ID/g) of almost 8 in the tumour tissue, whereas none of the other immunocytokines tested reached 6% ID/g at the tumor site (**Figures 2** **and** **6**). These results demonstrating that the "scDb C-terminus" conjugate performed better than all of the other immunocytokine formats tested, including the "Crab" format, which is surprising in light of the teachings of WO2018/069467 where the "Crab" format out-performed all of the other formats tested, including the "scDb C-terminus" format (**Figure 5**). When N-terminal and C-terminal fusion proteins comprising IL4 and a single-chain F8 diabody were compared in WO2018/069467, the N-terminal fusion protein outperformed the C-terminal fusion, i.e. the opposite of the results obtained here, making the present results even more unexpected (Table 1 of WO2018/069467). The conjugates tested in WO2018/069467 comprised interleukin-4 (IL4). This makes the present results even more surprising, as IL4 and IL2 are very similar in structure and molecular weight (14.9 and 15.5 kDa, respectively) and both belong to the hematopoietin family of cytokines.

It was further surprising that the "scDb C-terminus" format outperformed the "scDb X 2" format in tumour targeting, as this format also comprises a single-chain diabody but comprises two IL2 conjugated to the N- and C-terminus of the specific binding member, respectively.

A conjugate comprising a single-chain diabody and one IL2 linked e.g. to the C-terminus of the single-chain diabody therefore displays excellent tumour targeting ability.

In addition, unlike heterodimeric formats, the immunocytokines of the present invention can be expressed as a single chain polypeptide, for example as a single chain fusion protein. This format has the advantage of being easier to produce and purify since it consists of a single species and is expected to facilitate production of clinical-grade material.

The results obtained with the conjugate of the invention have significant therapeutic implications for improved targeting of IL2 to tumours. Conjugates of the invention may thus be used in the treatment of cancer.

In a first aspect, the invention therefore relates to a conjugate comprising IL2 and a single-chain diabody. The IL2 is preferably linked to the single-chain diabody by a peptide linker. Suitable linkers are described herein. The linkage may be at the C-terminus or the N-terminus of the single-chain diabody but most preferably is at the C-terminus of the single-chain diabody.

The conjugate preferably comprises only one IL2. Thus, where the IL2 is linked to the C-terminus of the single-chain diabody, the N-terminus of the single-chain diabody is preferably free. Similarly, where the IL2 is linked to the N-terminus of the single-chain diabody, the C-terminus of the single-chain diabody is preferably free. Preferably, the conjugate contains only one single-chain diabody.

Preferably, the single-chain diabody binds an extra-cellular matrix component associated with neoplastic growth and/or angiogenesis. For example, the single-chain diabody may bind fibronectin (e.g. domain ED-B or ED-A) or tenascin-C (e.g. domain A1).

The single-chain diabody may comprise an antigen binding site having the complementarity determining regions (CDRs) of antibody L19 set forth in SEQ ID NOs 4-9. The antigen binding site may comprise VH and/or VL domains of antibody L19 set forth in SEQ ID NOs 2 and 3, respectively. The single-chain diabody preferably comprises the L19 diabody amino acid sequence set forth in SEQ ID NO: 10. More preferably the single-chain diabody comprises or consist of the L19 single-chain diabody amino acid sequence set forth in SEQ ID NO: 11.

Other antibodies capable of binding to ECM proteins such as fibronectin, for example F8 (specific to ED-A), or F16 (specific to the A1 domain of tenascin-C) are known, and fragments of these antibodies, for example their CDRs, VH and/or VL domains, may be used in single-chain diabodies forming part of the conjugates of the invention.

Preferably, the conjugate has at least 70% sequence identity, more preferably one of at least 75%, 80%, 85%, 90%, 95%, 96%, 97%, 98%, 99% or 100%, sequence identity, to the amino acid sequence of L19-IL2 scDb C-terminus set out in SEQ ID NO: 12. Alternatively, the conjugate may have at least 70% sequence identity, more preferably one of at least 75%, 80%, 85%, 90%, 95%, 96%, 97%, 98%, 99% or 100%, sequence identity, to the amino acid sequence of IL2-L19 scDb N-terminus set out in SEQ ID NO: 13. The conjugate preferably comprises or consists of the amino acid sequence set forth in SEQ ID NO: 12, but may alternatively comprise or consist of the amino acid sequence set forth in SEQ ID NO: 13.

The invention also provides isolated nucleic acids encoding conjugates of the invention. An isolated nucleic acid may be used to express the conjugate of the invention, for example by expression in a bacterial, yeast, insect or mammalian host cell. The encoded nucleic acid will generally be provided in the form of a recombinant vector for expression. Host cells *in vitro* comprising such vectors are part of the invention, as is their use for expressing the fusion proteins, which may subsequently be purified from cell culture and optionally formulated into a pharmaceutical composition.

A conjugate or immunocytokine of the invention may be provided for example in a pharmaceutical composition, and may be employed for medical use as described herein, either alone or in combination with one or more further therapeutic agents.

In another aspect the invention relates to a conjugate as herein described for use in a method of treating cancer by targeting IL2 to the neovasculature *in vivo.*

In another aspect the invention relates to a method of treating cancer by targeting IL2 to the neovasculature in a patient, the method comprising administering a therapeutically effective amount of a conjugate as herein described to the patient.

### Brief Description of the Figures

**Figure 1** shows the structure of the IL2 conjugates tested. A: Diabody comprising the VH and VL domains of antibody L19 linked by a 5 amino acid linker sequence (GSSGG, GGSGG, GSADG, or GSAKG) wherein the IL2 is linked to the VL domains of diabody via a 15 amino acid linker. **B:** scFv₂ wherein IL2 is fused at its N-terminus via a linker, to the C-terminus of the VL domain of a single-chain variable fragment (scFv) molecule comprising the VH and VL domains of L19. Dimerisation of the L19 scFv results in the formation of the scFv L19-IL2 homodimer. **C:** a single-chain diabody molecule comprising the VH and VL domains of L19 wherein IL2 is linked to the N- and C-terminus of the diabody molecule (scDb x2). **D:** IL2 linked at its N- and C-terminus to scFv molecules comprising the VH and VL domains of antibody L19 (Crab). **E:** Single-chain diabody comprising the VH and VL domains of antibody L19 wherein IL2 is linked to the C-terminus of the diabody (scDb C-terminus). **F:** Single-chain diabody comprising the VH and VL domains of antibody L19 wherein IL2 is linked to the N-terminus of the diabody (scDb N-terminus).
**Figure 2** shows the results of comparative biodistribution analysis of different immunocytokine formats comprising L19 and IL2 in a F9 teratocarcinoma mouse tumour model (n = 3 per group). The percentage of the injected dose (ID) of the immunocytokine labeled with ¹²⁵I per gram of tissue (%ID/g) in tumor, blood and organs 24 Hours after administration is shown. **A:** Diabody GSSGG. **B:** Diabody GGSGG. **C:** Diabody GSADG. **D:** Diabody GSAKG. **E:** scFv₂. **F:** scDb X2. **G:** Crab. **H:** scDb C-terminus. All of the immunocytokine formats tested showed a preferential uptake in the tumor and favourable tumor-to-organ profile, with the highest tumor uptake seen with the scDb C-terminus format (%ID/g of about 7.7).
**Figure 3** shows the different immunocytokine formats tested for a conjugate comprising IL12 in WO2006/119897. **A:** scFv format. **B:** SIP format. **C:** dimeric format.
**Figure 4** shows the different immunocytokine formats tested for a conjugate comprising IL12 in WO2013/014149. **A:** dimeric format. **B:** format x 2. **C:** scDb N-terminus.
**Figure 5** shows the different immunocytokine formats tested for a conjugate comprising IL4 in WO2018/069467. **A:** scDb N-terminus. **B:** scDb C-terminus. **C:** Diabody N-terminus. **D:** Diabody C-terminus. **E:** Crab.
**Figure 6** shows the results of comparative biodistribution analysis of different immunocytokine formats comprising L19 and IL2 in a F9 teratocarcinoma mouse tumour model (n = 5 per group): scDb C-terminus, scDb N-terminus and scFv₂. The percentage of the injected dose (ID) of the immunocytokine labeled with ¹²⁵I per gram of tissue (%ID/g) in tumor, blood and organs 24 hours after administration is shown. All of the immunocytokine formats tested showed a preferential uptake in the tumor and favourable tumor-to-organ profile, with the highest tumor uptake seen with the scDb C-terminus format (%ID/g of about 7.8).

### Detailed Description

### Conjugate

Conjugates of the invention comprise IL2, and single-chain diabody.

The conjugate may be or may comprise a single-chain protein. When the conjugate is a single-chain protein, the entire protein can be expressed as a single polypeptide. For example, the conjugate may be a single-chain protein comprising IL2 and a single-chain diabody. The single-chain protein may be a fusion protein, for example a single-chain fusion protein comprising IL2 and a single-chain diabody. By "single-chain fusion protein" is meant a polypeptide that is a translation product resulting from the fusion of two or more genes or nucleic acid coding sequences into one open reading frame (ORF). The fused expression products of the two genes in the ORF may be conjugated by a peptide linker encoded in-frame. Suitable peptide linkers are described herein.

The conjugate preferably comprises only one IL2. The IL2 may be linked to the C-terminus or the N-terminus of the single-chain diabody but most preferably is linked to the C-terminus of the single-chain diabody. The linkage may be direct or may be indirect, for example via a peptide linker. Suitable linkers and ways of linking are disclosed herein. Where the IL2 is conjugated to the C-terminus of the single-chain diabody, the N-terminus of the single-chain diabody is preferably free. Similarly, where the IL2 is conjugated to the N-terminus of the single-chain diabody, the C-terminus of the single-chain diabody is preferably free. "Free" in this context refers to the N- or C-terminus not being linked or otherwise conjugated to another moiety, such as IL2.

### Single-chain diabody

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen-binding site: antigen-binding sites are formed by the association of the first domain of one polypeptide within the multimer with the second domain of another polypeptide within the multimer (WO94/13804; Holliger and Winter, 1997; Holliger *et al.,* 1993).

In a diabody a heavy chain variable domain (VH) is connected to a light chain variable domain (VL) on the same polypeptide chain. The VH and VL domains are connected by a peptide linker that is too short to allow pairing between the two domains (generally around 5 amino acids). This forces paring with the complementary VH and VL domains of another chain. An example of this format is shown in Figure 1A. The diabody-based IL2 conjugates tested showed lower tumour targeting that the single-chain diabody-based IL2 conjugates. The conjugate of the invention therefore preferably comprises a single-chain diabody. In a single-chain diabody two sets of VH and VL domains are connected together in sequence on the same polypeptide chain. For example, the two sets of VH and VL domains may be assembled in a single-chain sequence as follows:
(VH-VL)--(VH-VL), where the brackets indicate a set.

In the single-chain diabody format each of the VH and VL domains within a set is connected by a short or 'non-flexible' peptide linker. This type of peptide linker sequence is not long enough to allow pairing of the VH and VL domains within the set. Generally a short or 'non flexible' peptide linker is around 5 amino acids.

The two sets of VH and VL domains are connected as a single-chain by a long or 'flexible' peptide linker. This type of peptide linker sequence is long enough to allow pairing of the VH and VL domains of the first set with the complementary VH and VL domains of the second set. Generally a long or 'flexible' linker is 15 to 20 amino acids.

Single-chain diabodies have been previously generated (Konterman & Muller, 1999). A bispecific single-chain diabody has been used to target adenovirus to endothelial cells (Nettelbeck *et al.,* 2001).

A single-chain diabody is bivalent i.e. has two antigen-binding sites, each comprising an antibody light chain variable region (VL) and an antibody heavy chain variable region (VH). An "antigen-binding site" describes the part of the single-chain diabody which comprises the area which specifically binds to and is complementary to part or all of an antigen. Where an antigen is large, the single-chain diabody may only bind to a particular part of the antigen, which part is termed an epitope.

The antigen-binding sites of the single-chain diabody may be identical or different but preferably are identical. Each of the antigen-binding sites in the single-chain diabody may bind the same antigen or epitope. This can be achieved by providing two identical antigen-binding sites such as two identical VH-VL domain pairs, or by providing two different antigen-binding sites, for example comprising different VH and VL domains, which nevertheless both bind the same antigen or epitope. Alternatively, the single-chain diabody may be bispecific. By 'bispecific" we mean that each of the antigen-binding sites binds a different antigen. Optionally, two antigen-binding sites may bind two different antigens mentioned herein, e.g. two different antigens of the extracellular matrix, or two different domains of a particular antigen (e.g. fibronectin or tenascin-C).

The single-chain diabody may bind an extra-cellular matrix (ECM) component associated with neoplastic growth and/or angiogenesis. The binding may be specific. The term "specific" may be used to refer to the situation in which one member of a specific binding pair will not show any significant binding to molecules other than its specific binding partner(s). The term is also applicable where e.g. an antigen-binding site is specific for a particular epitope that is carried by a number of antigens, in which case the single-chain diabody carrying the antigen-binding site will be able to bind to the various antigens carrying the epitope. Preferably the single-chain diabody binds fibronectin. Fibronectin is an antigen subject to alternative splicing, and a number of alternative isoforms of fibronectin are known, including alternatively spliced isoforms A-FN and B-FN, comprising domains ED-A or ED-B respectively, which are known markers of angiogenesis. The single-chain diabody may selectively bind to isoforms of fibronectin selectively expressed in the neovasculature. An antigen-binding site in the single-chain diabody may bind fibronectin isoform A-FN, e.g. it may bind domain ED-A (extra domain A). In a preferred embodiment, an antigen-binding site in the single-chain diabody binds fibronectin isoform B-FN, e.g. it may bind ED-B (extra domain B).

Fibronectin Extra Domain-A (EDA or ED-A) is also known as ED, extra type III repeat A (EIIIA) or EDI. The sequence of human ED-A has been published by Kornblihtt et al. (1984), Nucleic Acids Res. 12, 5853-5868 and Paolella et al. (1988), Nucleic Acids Res. 16, 3545-3557. The sequence of human ED-A is also available on the SwissProt database as amino acids 1631-1720 (Fibronectin type-III 12; extra domain 2) of the amino acid sequence deposited under accession number P02751. The sequence of mouse ED-A is available on the SwissProt database as amino acids 1721-1810 (Fibronectin type-III 13; extra domain 2) of the amino acid sequence deposited under accession number P11276.

The ED-A isoform of fibronectin (A-FN) contains the Extra Domain-A (ED-A). The sequence of the human A-FN can be deduced from the corresponding human fibronectin precursor sequence which is available on the SwissProt database under accession number P02751. The sequence of the mouse A-FN can be deduced from the corresponding mouse fibronectin precursor sequence which is available on the SwissProt database under accession number P11276. The A-FN may be the human ED-A isoform of fibronectin. The ED-A may be the Extra Domain-A of human fibronectin.

ED-A is a 90 amino acid sequence which is inserted into fibronectin (FN) by alternative splicing and is located between domain 11 and 12 of FN (Borsi *et al.,* 1987). ED-A is mainly absent in the plasma form of FN but is abundant during embryogenesis, tissue remodelling, fibrosis, cardiac transplantation and solid tumour growth.

Fibronectin isoform B-FN is one of the best known markers angiogenesis (WO1997/045544). An extra domain "ED-B" of 91 amino acids is found in the B-FN isoform and is identical in mouse, rat, rabbit, dog and man. B-FN accumulates around neovascular structures in aggressive tumours and other tissues undergoing angiogenesis, such as the endometrium in the proliferative phase and some ocular structures in pathological conditions, but is otherwise undetectable in normal adult tissues.

The single-chain diabody may bind tenascin-C. Tenascin-C is a large hexameric glycoprotein of the extracellular matrix which modulates cellular adhesion. It is involved in processes such as cell proliferation and cell migration and is associated with changes in tissue architecture as occurring during morphogenesis and embryogenesis as well as under tumourigenesis or angiogenesis. Several isoforms of tenascin-C can be generated as a result of alternative splicing which may lead to the inclusion of (multiple) domains in the central part of this protein, ranging from domain A1 to domain D (Borsi L et al Int J Cancer 1992; 52:688-692, Carnemolla B et al. Eur J Biochem 1992; 205:561-567, WO2006/050834). An antigen-binding site in the single-chain diabody may bind tenascin-C domain A1.

The single-chain diabody may comprise an antigen-binding site having the complementarity determining regions (CDRs), or the VH and/or VL domains of an antibody capable of specifically binding to an antigen of interest, for example, one or more CDRs or VH and/or VL domains of an antibody capable of specifically binding to an antigen of the ECM. The antigen may be an antigen preferentially expressed by cells of a tumour or tumour neovasculature or associated with the ECM. Such antigens include fibronectin and tenascin C, as described above.

Thus, the single-chain diabody may comprise an antigen-binding site of the antibody F8, the antibody L19 or the antibody F16, which have all been shown to bind specifically to ECM antigens. The single-chain diabody may comprise an antigen-binding site having one, two, three, four, five or six CDR's, or the VH and/or VL domains of antibody F8, L19 or F16.

L19 is a human monoclonal scFv specific alternatively spliced ED-B domain of fibronectin and has been previously described (WO1999/058570; WO2006/119897). F8 is a human monoclonal scFv antibody fragment specific to the alternatively spliced ED-A domain of fibronectin and has been previously described (WO2008/120101; Villa *et al.,* 2008). F16 is a human monoclonal scFv specific to the A1 domain of Tenascin C and has been previously described (WO2006/050834).

An antigen-binding site may comprise one, two, three, four, five or six CDRs of antibody L19. Amino acid sequences of the CDRs of L19 are:
SEQ ID NO:4 (CDR1 VH);
SEQ ID NO:5 (CDR2 VH);
SEQ ID NO:6 (CDR3 VH);
SEQ ID NO:7 (CDR1 VL);
SEQ ID NO:8 (CDR2 VL), and/or
SEQ ID NO:9 (CDR3 VL).

SEQ ID NOs 4-6 are the amino acid sequences of the VH CDR regions (1-3, respectively) of the human monoclonal antibody L19. SEQ ID NOs 7-9 are the amino acid of the VL CDR regions (1-3, respectively) of the human monoclonal antibody L19. The amino acid sequence of the VH and VL domains of antibody L19 correspond to SEQ ID NOs 2 and 3, respectively.

An antigen-binding site may comprise one, two, three, four, five or six CDRs of antibody F8. Amino acid sequences of the CDRs of F8 are:
SEQ ID NO: 56 (CDR1 VH);
SEQ ID NO: 57 (CDR2 VH);
SEQ ID NO: 58 (CDR3 VH);
SEQ ID NO: 59 (CDR1 VL);
SEQ ID NO: 60 (CDR2 VL), and/or
SEQ ID NO: 61 (CDR3 VL).

SEQ ID NOs 56-58 are the amino acid sequences of the VH CDR regions (1-3, respectively) of the human monoclonal antibody F8. SEQ ID NOs 59-61 are the amino acid of the VL CDR regions (1-3, respectively) of the human monoclonal antibody F8. The amino acid sequence of the VH and VL domains of antibody F8 correspond to SEQ ID NO: 54 and SEQ ID NO: 55, respectively.

An antigen-binding site may comprise one, two, three, four, five or six CDRs of antibody F16. Amino acid sequences of the CDRs of F16 are:
SEQ ID NO: 65 (CDR1 VH);
SEQ ID NO: 66 (CDR2 VH);
SEQ ID NO: 67 (CDR3 VH);
SEQ ID NO: 68 (CDR1 VL);
SEQ ID NO: 69 (CDR2 VL), and/or
SEQ ID NO: 70 (CDR3 VL).

SEQ ID NOs 65-67 are the amino acid sequences of the VH CDR regions (1-3, respectively) of the human monoclonal antibody F16. SEQ ID NOs 68-70 are the amino acid of the VL CDR regions (1-3, respectively) of the human monoclonal antibody F16. The amino acid sequence of the VH and VL domains of antibody F16 correspond to SEQ ID NO: 63 and SEQ ID NO: 64, respectively.

The conjugate of the invention preferably comprises IL2 joined to a single-chain diabody, for example a single-chain diabody comprising the VH and VL domains of antibody L19, F8, or F16, preferably antibody L19.

A single-chain diabody according to the invention may have a VH domain having at least 70%, more preferably one of at least 75%, 80%, 85%, 90%, 95%, 96%, 97%, 98%, 99% or 100%, sequence identity to the L19 VH domain amino acid sequence SEQ ID NO: 2, the F8 VH domain amino acid sequence SEQ ID NO: 54, or the F16 VH domain amino acid sequence SEQ ID NO: 63.

A single-chain diabody according to the invention may have a VL domain having at least 70%, more preferably one of at least 75%, 80%, 85%, 90%, 95%, 96%, 97%, 98%, 99% or 100%, sequence identity to the L19 VL domain amino acid sequence SEQ ID NO: 3, the F8 amino acid sequence SEQ ID NO: 55 or the F16 amino acid sequence SEQ ID NO: 64.

Sequence identity is commonly defined with reference to the algorithm GAP (Wisconsin GCG package, Accelerys Inc, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul *et al.* (1990) supra, generally employing default parameters. In particular, the psi-Blast algorithm (Nucl. Acids Res. (1997) 25 3389-3402) may be used.

Variants of these VH and VL domains and CDRs may also be employed in antibody molecules for use in conjugates as described herein. Suitable variants can be obtained by means of methods of sequence alteration, or mutation, and screening.

Particular variants for use as described herein may include one or more amino acid sequence alterations (addition, deletion, substitution and/or insertion of an amino acid residue), maybe less than about 20 alterations, less than about 15 alterations, less than about 10 alterations or less than about 5 alterations, 4, 3, 2 or 1.

Alterations may be made in one or more framework regions and/or one or more CDRs. In particular, alterations may be made in VH CDR1, VH, CDR2 and/or VH CDR3.

The single-chain diabody may comprise the sequence set forth in SEQ ID NO: 10, or sequence which has at least 70%, more preferably one of at least 75%, 80%, 85%, 90%, 95%, 96%, 97%, 98%, 99% or 100%, sequence identity to the amino acid sequence set forth in SEQ ID NO: 10.

The amino acid sequence of the L19 single-chain diabody is found in SEQ ID NO: 11. The L19 single-chain diabody may comprise or consist the amino acid sequence of SEQ ID NO: 11.

A single-chain diabody for use in the invention may have at least 70%, more preferably one of at least 75%, 80%, 85%, 90%, 95%, 96%, 97%, 98%, 99% or 100%, sequence identity to the amino acid sequence of the L19 single-chain diabody set forth in SEQ ID NO: 11.

Alternatively, a single-chain diabody for use in the invention may have at least 70%, more preferably one of at least 75%, 80%, 85%, 90%, 95%, 96%, 97%, 98%, 99% or 100%, sequence identity to the amino acid sequence of the F8 single-chain diabody set forth in SEQ ID NO: 62, or the F16 single-chain diabody set forth in SEQ ID NO: 71.

### Linkers

The single-chain diabody and IL2 may be connected to each other directly, for example through any suitable chemical bond or through a linker, for example a peptide linker, but preferably are connected by a peptide linker. The peptide linker may be a short (2-30, preferably 10-20) residue stretch of amino acids. Suitable examples of peptide linker sequences are known in the art. One or more different linkers may be used. The linker may be about 15 amino acids in length. An example of a suitable linker is (G₄S)₃ (SEQ ID NO: 26).

The chemical bond may be, for example, a covalent or ionic bond. Examples of covalent bonds include peptide bonds (amide bonds) and disulphide bonds. For example, the single-chain diabody and IL2 may be covalently linked. For example, by peptide bonds (amide bonds). Thus, the single-chain diabody and IL2 may be produced (secreted) as a single-chain polypeptide. The single-chain diabody and IL2 may also be connected directly, for example through any suitable chemical bond, or through a linker, for example a peptide linker. Examples of individual components which may be linked within the single-chain diabody are VH and VL sequences.

For example, the first and second set of VH and VL sequences of the single-chain diabody are preferably connected by a flexible peptide linker. By "flexible" is meant a linker sequence that is long enough to allow pairing of the VH and VL domains of the first set with the complementary VH and VL domains of the second set. Generally, a long or 'flexible' linker is at least 10 amino acids, preferably 10 to 20 amino acid. Single-chain diabodies have been previously generated and described by Kontermann, R. E., and Muller, R. (1999), J. Immunol. Methods 226: 179-188. An example of such a linker is GSLDGAGGSAGADGG (SEQ ID NO: 25). Preferably the VH-VL sequences within each set are connected by a 'non-flexible' linker. By a 'non-flexible' linker is meant a peptide linker sequence that is not long enough to allow pairing of the VH and VL domains. An example of a short linker sequence is GSSGG (SEQ ID NO: 21).

### Interleukin-2 (IL2)

The conjugate of the invention comprises IL2. The IL2 may be derived from any animal, e.g. human, rodent (e.g. rat, mouse), horse, cow, pig, sheep, dog, etc. Human IL2 is preferred in conjugates for administration to humans. The amino acid sequence of human IL2 is set out in SEQ ID NO: 1. The conjugate of the invention preferably comprises a single IL2 polypeptide.

Typically, the IL2 has at least 70%, more preferably one of at least 75%, 80%, 85%, 90%, 95%, 96%, 97%, 98%, 99% or 100%, sequence identity to the amino acid sequence shown in SEQ ID NO: 1.

IL2 in conjugates of the invention retains a biological activity of IL2, e.g. an ability to promote proliferation and differentiation of activated T and B lymphocytes and natural killer (NK) cells, induce cytotoxic T cell (CTL) activity, and/or NK/lymphokine-activated killer (LAK) cell antitumor cytotoxicity.

The IL2 may be conjugated to either the C- or the N- terminus of the single-chain diabody.

### Methods of treatment

A conjugate according to the invention may be used in a method of treatment of the human or animal body, such as a method of treatment (which may include prophylactic treatment) of a cancer in a patient (typically a human patient) comprising administering the conjugate to the patient.

Accordingly, such aspects of the invention provide methods of treatment comprising administering a conjugate of the invention, pharmaceutical compositions comprising such a conjugate for the treatment of cancer in a patient, and a method of making a medicament or pharmaceutical composition comprising formulating the conjugate of the present invention with a physiologically acceptable carrier or excipient.

Thus, a conjugate of the invention may be for use in a method of treating cancer by targeting IL2 to the tumour neovasculature *in vivo.* Also contemplated is a method of treating cancer by targeting IL2 to the neovasculature in a patient, the method comprising administering a therapeutically effective amount of a conjugate of the invention to the patient. Also provided is the use of a conjugate of the invention for the manufacture of a medicament for the treatment of cancer.

Conditions treatable using the conjugate as described herein include cancer, other tumours and neoplastic conditions. Treatment may include prophylactic treatment.

Cancers suitable for treatment as described herein include any type of solid or non-solid cancer or malignant lymphoma and especially malignant melanoma, Merkel-cell carcinoma, renal cell cancer, leukaemia (e.g. acute myeloid leukaemia), non-small cell lung cancer (NSCLC), oligometastatic solid tumors, liver cancer, lymphoma, sarcomas, skin cancer, bladder cancer, breast cancer, uterine cancer, ovarian cancer, prostate cancer, lung cancer, colorectal cancer, cervical cancer, head and neck cancer, oesophageal cancer, pancreatic cancer, stomach cancer and cerebral cancer. Cancers may be familial or sporadic. Cancers may be metastatic or non-metastatic.

Preferably, the cancer is a cancer selected from the group of malignant melanoma, Merkel-cell carcinoma, renal cell cancer, acute myeloid leukaemia (AML), non-small cell lung cancer (NSCLC), colon carcinoma and oligometastatic solid tumors.

The cancer may express an isoform of fibronectin comprising domain ED-A or ED-B, or alternatively spliced tenascin-C comprising for example domain A1. Preferably the cancer expresses the ED-B isoform of fibronectin.

Expression of the ED-B isoform of fibronectin has been reported in a number of different cancers.

### Pharmaceutical compositions

A further aspect of the invention relates to a pharmaceutical composition comprising at least one conjugate of the invention and optionally a pharmaceutically acceptable excipient.

Pharmaceutical compositions of the invention typically comprise a therapeutically effective amount of a conjugate according to the invention and optionally auxiliary substances such as pharmaceutically acceptable excipient(s). Said pharmaceutical compositions are prepared in a manner well known in the pharmaceutical art. A carrier or excipient may be a liquid material which can serve as a vehicle or medium for the active ingredient. Suitable carriers or excipients are well known in the art and include, for example, stabilisers, antioxidants, pH-regulating substances, controlled-release excipients. The pharmaceutical preparation of the invention may be adapted, for example, for parenteral use and may be administered to the patient in the form of solutions or the like.

Compositions comprising the conjugate of the invention may be administered to a patient. Administration is preferably in a "therapeutically effective amount", this being sufficient to show benefit to the patient. Such benefit may be at least amelioration of at least one symptom. The actual amount administered, and rate and time-course of administration, will depend on the nature and severity of what is being treated. Prescription of treatment, e.g. decisions on dosage etc, is within the responsibility of general practitioners and other medical doctors. Treatments may be repeated at daily, twice-weekly, weekly, or monthly intervals at the discretion of the physician

Conjugates of the invention may be administered to a patient in need of treatment via any suitable route, usually by injection into the bloodstream and/or directly into the site to be treated, e.g. tumour or tumour vasculature. The precise dose and its frequency of administration will depend upon a number of factors, the route of treatment, the size and location of the area to be treated (e.g. tumour).

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included

For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A pharmaceutical composition comprising a conjugate of the invention may be administered alone or in combination with other treatments, either simultaneously or sequentially dependent upon the condition to be treated. Other treatments may include the administration of suitable doses of pain relief drugs such as non-steroidal anti-inflammatory drugs (e.g. aspirin, paracetamol, ibuprofen or ketoprofen) or opiates such as morphine, or anti-emetics, as well as a second anti-cancer therapy.

### Kits

Another aspect of the invention provides a therapeutic kit for use in the treatment of cancer comprising a conjugate of the invention. The components of a kit are preferably sterile and in sealed vials or other containers. A kit may further comprise instructions for use of the components in a method described herein. The components of the kit may be comprised or packaged in a container, for example a bag, box, jar, tin or blister pack.

### Nucleic acids, vectors, host cells and methods of production

Also provided is an isolated nucleic acid molecule encoding a conjugate according to the invention. Nucleic acid molecules may comprise DNA and/or RNA and may be partially or wholly synthetic.

Further provided are constructs in the form of plasmids, vectors, transcription or expression cassettes which comprise such nucleic acids. Suitable vectors can be chosen or constructed, containing appropriate regulatory sequences, including promoter sequences, terminator sequences, polyadenylation sequences, enhancer sequences, marker genes and other sequences as appropriate. Vectors may be plasmids e.g. phagemid, or viral e.g. 'phage, as appropriate. For further details, see, for example, Sambrook & Russell (2001) Molecular Cloning: a Laboratory Manual: 3rd edition, Cold Spring Harbor Laboratory Press. Many known techniques and protocols for manipulation of nucleic acid, for example in the preparation of nucleic acid constructs, mutagenesis, sequencing, introduction of DNA into cells and gene expression, and analysis of proteins, are described in detail in Ausubel et al. (1999) 4th eds., Short Protocols in Molecular Biology: A Compendium of Methods from Current Protocols in Molecular Biology, John Wiley & Sons.

A recombinant host cell that comprises one or more constructs as described above is also provided. Suitable host cells include bacteria, mammalian cells, plant cells, filamentous fungi, yeast and baculovirus systems and transgenic plants and animals.

A conjugate according to the present invention may be produced using such a recombinant host cell. The production method may comprise expressing a nucleic acid or construct as described above. Expression may conveniently be achieved by culturing the recombinant host cell under appropriate conditions for production of the conjugate. Following production, the conjugate may be isolated and/or purified using any suitable technique, and then used as appropriate. The conjugate may be formulated into a composition including at least one additional component, such as a pharmaceutically acceptable excipient.

Systems for cloning and expression of a polypeptide in a variety of different host cells are well known. The expression of antibodies, including conjugates thereof, in prokaryotic cells is well established in the art. For a review, see for example Pluckthun (1991), Bio/Technology 9: 545-551. A common bacterial host is *E.coli.*

Expression in eukaryotic cells in culture is also available to those skilled in the art as an option for production of conjugates for example Chadd et al. (2001), Current Opinion in Biotechnology 12: 188-194); Andersen et al. (2002) Current Opinion in Biotechnology 13: 117; Larrick & Thomas (2001) Current Opinion in Biotechnology 12:411-418. Mammalian cell lines available in the art for expression of a heterologous polypeptide include Chinese hamster ovary (CHO) cells, HeLa cells, baby hamster kidney cells, NS0 mouse melanoma cells, YB2/0 rat myeloma cells, human embryonic kidney cells, human embryonic retina cells and many others.

A method comprising introducing a nucleic acid or construct disclosed herein into a host cell is also described. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. Introducing nucleic acid in the host cell, in particular a eukaryotic cell may use a viral or a plasmid based system. The plasmid system may be maintained episomally or may be incorporated into the host cell or into an artificial chromosome. Incorporation may be either by random or targeted integration of one or more copies at single or multiple loci. For bacterial cells, suitable techniques may include calcium chloride transformation, electroporation and transfection using bacteriophage.

The nucleic acid or construct may be integrated into the genome (e.g. chromosome) of the host cell. Integration may be promoted by inclusion of sequences that promote recombination with the genome, in accordance with standard techniques.

Further aspects and embodiments of the invention will be apparent to those skilled in the art given the present disclosure including the following experimental exemplification.

All documents mentioned in this specification are incorporated herein by reference in their entirety.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Other aspects and embodiments of the invention provide the aspects and embodiments described above with the term "comprising" replaced by the term "consisting of" or "consisting essentially of", unless the context dictates otherwise.

Certain aspects and embodiments of the invention will now be illustrated by way of example and with reference to the figures described above.

### Examples

### Example 1 - Cloning of L19-IL2 immunocytokines

The below examples describe the production and characterisation of nine L19-IL2 immunocytokines which differ in format and/or sequence, and compare these formats with regard to their ability to specifically target tumours in a mouse cancer model, thereby demonstrating the superiority of the single-chain diabody format as claimed herein for *in vivo* targeting.

### 1.1 Cloning of four L19 diabody-IL2 conjugates comprising different VH-VL domain linker sequences

### 1.1.1 Cloning of the L19 diabody-IL2 conjugate with a GSSGG VH-VL domain linker sequence

DNA fragments encoding the L19 diabody comprising different VH-VL domain linker sequences were prepared: GSSGG (composed of two polar serines and neutral glycines), GGSGG (composed of the polar amino acid serine and neutral glycines), GSADG (negatively charged under physiological conditions of pH 7.4) and GSAKG (positively charged under physiological conditions of pH 7.4). Differently charged linker sequences were tested to determine which linker would exhibit the best performance.

The DNA fragment encoding the L19 diabody comprising the GSSGG VH-VL domain linker sequence was cloned by PCR-amplification of the L19 gene using primers HindIIISIP (SEQ ID NO: 50) and L19Linker (SEQ ID NO: 51). The DNA sequence encoding the IL2 was cloned from the IL2 gene using primers LinkerIL2 (SEQ ID NO: 52) and IL2stopNotl (SEQ ID NO: 53). The two DNA fragments (L19 antibody and IL2) were assembled by means of PCR, amplified using primers HindIIISIP and IL2stopNotl, double digested with HindIII/NotI and cloned into a pcDNA 3.1 (+) vector. The amino acid sequence of the L19 diabody-IL2 polypeptide with the GSSGG VH-VL domain linker is shown is SEQ ID NO: 16. A schematic representation of the L19 diabody-IL2 conjugate is shown in **Figure 1A****.**

### 1.1.2 Cloning of diabody-IL2 conjugates with different VH-VL domain linker sequences

Three further diabody-IL2 conjugates were prepared by inserting linker sequences GGSGG, GSADG and GSAKG between the heavy and light chain variable domains of the L19 diabody-IL2 conjugate prepared in 1.1.1. The L19 diabody-IL2 conjugates were prepared by means of PCR assembly of a fragment "A" (encoding the L19 heavy chain variable domain), and a fragment "B" (encoding the L19 light chain variable domain and the IL2 payload). Fragments "A" and "B" were amplified from the L19 diabody-IL2 molecule prepared in 1.1.1 using the primers listed in the Tables 1 and 2 below.

**Table 1: Primers used for PCR amplification of "A" fragments of L19 diabody-IL2.**

| Fragment A | | |
|---|---|---|
| Clone | **Forward Primer** | **Backward Primer** |
| GGSGG | TAATACGACTCACTATAGGG (SEQ ID NO 27) | CACCGCCTGATCCCCCACTCGAGACGGTGACCAGG GT (SEQ ID NO 28) |
| GSADG | TAATACGACTCACTATAGGG (SEQ ID NO 27) | CGTCTGCTGACCCACTCGAGACGGTGACCAGGGTT CCC (SEQ ID NO 29) |
| GSAKG | TAATACGACTCACTATAGGG (SEQ ID NO 27) | ACCTTTTGCTGACCCACTCGAGACGGTGACCAGGG TTCCC (SEQ ID NO 30) |

**Table 2: Primers used for PCR amplification of "B" fragments of L19 diabody-IL2.**

| Fragment B | | |
|---|---|---|
| Clone | **Forward Primer** | **Backward Primer** |
| GGSGG | TCTCGAGTGGGGATCAGGCGGTGAA ATTGTGTTGACGCAG (SEQ ID NO 31) | TAGAAGGCACAGTCGAGG (SEQ ID NO 32) |
| GSADG | CCGTCTCGAGTGGGTCAGCAGACGGT GAAATTGTGTTGACGCAGTCT (SEQ ID NO 33) | TAGAAGGCACAGTCGAGG (SEQ ID NO 32) |
| GSAKG | CCGTCTCGAGTGGGTCAGCAAAAGGT GAAATTGTGTTGACGCAGTCT (SEQ ID NO 34) | TAGAAGGCACAGTCGAGG (SEQ ID NO 32) |

The "A" and "B" fragments were then PCR-assembled, PCR-amplified and double digested with Hindlll/Notl-HF and cloned into the double digested vector pcDNA 3.1 (+).

The resulting plasmids were amplified and used for cell transfection. The amino acid sequences of the L19 diabody-IL2 polypeptides with the GGSGG, GSADG and GSAKG linker sequences are set out in SEQ ID NOs 17 to 19. A schematic representation of the L19 diabody-IL2 conjugates is shown in **Figure 1A****.**

### 1.2 Cloning of scFv L19-IL2 (scFv₂)

The scFv L19-IL2 (scFv₂) is an immunocytokine, consisting of human IL2 fused at its N-terminus, via a 17 amino acid linker, to the C-terminus of antibody L19 in scFv format (see Figure 1B). The production and purification of scFv L19-IL2 was performed as described in WO01/062298. Dimerisation via the L19 scFv results in the formation of scFv L19-IL2 homodimers. The amino acid sequence of the scFv L19-IL2 polypeptide is set out in SEQ ID NO: 15. A schematic representation of the scFv L19-IL2 conjugate is shown in **Figure 1B****.**

### 1.3 Cloning of the L19 scDb with IL2 conjugated to its C- and N-termini ("scDb X 2")

The L19 scDb X2 immunocytokine (IL2-L19L19-IL2) coding sequence was generated using the previously cloned the scDb C-terminus (L19L19-IL2) and the scDb N-terminus (IL2-L19L19) as starting material.

The vector containing the sequence Nhel (restriction site)-IL2-L19-Hindlll (restriction site)-L19 was digested by NheI/HindIII in order to obtain the IL2-L19 DNA fragment. At the same time, the vector containing the sequence Nhel (restriction site)-L19- HindIII (restriction site)-L19-IL2 was digested by NheI/HindIII, in order to remove the first L19 moiety and replace it with Nhel (restriction site)-IL2-L19-HindIII (restriction site). By doing so, the full length scDb X2 (IL2-L19L19-IL2) was obtained.

The resulting plasmids were amplified and used for cell transfection. The amino acid sequence of above scDb X2 polypeptide is set out in SEQ ID NO: 20. A schematic representation of the L19 scDb X2 conjugate is shown in **Figure 1C****.**

### 1.4 Cloning of IL2 conjugated at its C-terminus and at its N-terminus to two scFv's ("Crab")

The "Crab" immunocytokine L19-IL2-L19 coding sequence was generated using L19-IL2 as template.

A first DNA fragment was amplified using primers HindLead> and DP47G4S2.5< (SEQ ID NO: 47). A second DNA fragment was amplified using primers G4S2.5VL> (SEQ ID NO: 48) and IL2G4S3Bam< (SEQ ID NO: 43). The two intermediate fragments were PCR-assembled, PCR-amplified using primers HindLead> and IL2G4S3Bam<, double digested with HindIII/BamHI and cloned into a pcDNA 3.1 (+) vector (resulting in L19-IL2).

A third DNA fragment was amplified using primers BamG4S3L19> (SEQ ID NO: 44) and DP47G4S2.5<. A fourth DNA fragment was amplified using primers G4S2.5VL> and L19StopNot< (SEQ ID NO: 45). The two intermediate fragments were PCR-assembled, PCR-amplified using primers BamG4S3L19> and L19StopNot<, double digested with BamHI/Notl HF and cloned into the previously generated vector, resulting in the full length L19-IL2-L19 "Crab" molecule. The resulting plasmids were amplified and used for cell transfection. The amino acid sequence of L19-IL2-L19 polypeptide is set out in SEQ ID NO: 14. A schematic representation of the L19 "Crab" conjugate is shown in **Figure 1D****.**

### 1.5 Cloning of the single-chain diabody (scDb) C-terminal fusion protein (L19L19-IL2)

The scDb C-terminal fusion protein coding sequence was generated using L19-IL2 as template.

The L19 gene was PCR amplified using primers LnkDP47> (SEQ ID NO: 39) and L19G4S3< (SEQ ID NO: 42). The IL2 gene was amplified using primers G4S3IL2> (SEQ ID NO: 41) and IL2StopNot< (SEQ ID NO: 46). The two intermediate fragments were PCR-assembled, PCR-amplified using primers HindLnk> (SEQ ID NO: 40) and IL2StopNot<, double digested with Hindlll/Notl-HF and cloned into a pcDNA 3.1 (+) vector.

A second L19 gene was PCR amplified using primers NheLead> (SEQ ID NO: 35) and L19Hind< (SEQ ID NO: 38), double digested by Nhel/Not-HF and inserted into the previously generated vector resulting into the full length L19L19-IL2.

The resulting plasmids were amplified and used for cell transfection. The amino acid sequence of L19L19-IL2 polypeptide is set out in SEQ ID NO: 12. A schematic representation of the L19 scDb C-terminal fusion protein is shown in **Figure 1E****.**

### 1.6 Cloning of the single-chain diabody (scDb) N-terminal fusion protein (IL2-L19L19)

The scDb N-terminal fusion protein (IL2-L19L19) coding sequence has been generated using L19-IL2 as template.

The IL2 gene was amplified using primers LeadIL2> (SEQ ID NO: 49) and IL2G4S3< (SEQ ID NO: 36). The L19 gene was PCR amplified from G4S3L19> (SEQ ID NO: 37) and L19Hind< (SEQ ID NO: 38). The two intermediate fragments were PCR-assembled, PCR-amplified using primers NheLead> (SEQ ID NO: 35) and L19Hind<, double digested with Nhel/Hindlll and cloned into a pcDNA 3.1 (+) vector.

A second L19 gene was PCR amplified a first time using primers LnkDP47> (SEQ ID NO: 39) and L19StopNot< (SEQ ID NO: 45) and a second time using primers HindLnk> (SEQ ID NO: 40) and L19StopNot<. The DNA fragment was subsequently double digested using Hindlll/Not-HF and inserted into the previously generated vector resulting in the full length IL2-L19L19.

The resulting plasmids were amplified and used for cell transfection. The amino acid sequence of the scDb N-terminal fusion protein is set out in SEQ ID NO: 13. A schematic representation of the L19 scDb N-terminal fusion protein is shown in **Figure 1F****.**

### Example 2 - Expression and purification of L19-IL2 immunocytokines

### 2.1 Cell culture and transfection

Transfected CHO-S cells (Chinese Hamster Ovary) were cultured in suspension in PowerCHO-2CD medium, supplemented with Ultraglutamine-1, HT-supplement and an antibiotic-antimycotic.

### 2.2 Expression and purification of L19-IL2 immunocytokines

The nine L19-IL2 immunocytokines described in Example 1 above were expressed using transient gene expression in CHO-S cells. For 1 mL of production 4 × 10⁶ CHO-S cells in suspension were centrifuged and resuspended in 1 mL ProCHO4 medium. 0.625 *µ*g of plasmid DNAs followed by 2.5 *µ*g polyethylene imine (PEI; 1 mg/mL solution in water at pH 7.0) per million cells were then added to the cells and gently mixed. The transfected cell cultures were incubated in a shaker incubator at 31°C for 6 days. The L19-IL2 immunocytokines were purified from the cell culture medium by protein A affinity chromatography and then dialyzed against PBS.

### Example 3 - characterization of the L19-IL2 immunocytokines

### 3.1 SDS-PAGE and size-exclusion chromatography

The purified immunocytokines prepared as described in **Example 2** were characterized by SDS-PAGE and size-exclusion chromatography. SDS-PAGE was performed with 10% gels under reducing and non-reducing conditions. Purified clones were analyzed by size-exclusion chromatography on a Superdex 200 increase 10/300 GL column on an AKTA FPLC.

SDS-PAGE characterization confirmed the expected molecular weight of the fusion proteins: around 80 kDa for the clones bearing a modified linker (based on non-covalent homodimeric diabody, Figure 1A), 66 kDa for the scDb fusion proteins (Figures 1E and 1F) and the crab format (Figure 1D) and 80 kDa for the fusion protein bearing one IL2 at both ends (scDb X 2, Figure 1C). The proteins exhibited a main peak of the expected elution volume in gel-filtration.

### 3.2 Affinity measurements

Affinity measurements were performed by surface plasmon resonance using a BIAcore X100 instrument using a fibronectin 7B89 domain coated CM5 chip. Samples were injected as serial-dilutions, in a concentration range from 1mM to 250nM. Regeneration of the chip was performed using 10 mM HCI. The BIAcore analysis confirmed the ability of the L19 antibody in the nine L19-IL2 immunocytokines to recognize the fibronectin 7B89 domain.

### Example 4 - Immunofluorescence and biodistribution experiments

### 4.1 Tumor implantation

The murine F9 teratocarcinoma tumor cell line was used to generate the syngeneic tumor model. F9 cells were cultured on 0.1% gelatin-coated tissue culture flasks in DMEM medium supplemented with 10% FCS. F9 tumor cells (12 × 10⁶ cells resuspended in 150 *µ*L of HBSS buffer) were then implanted subcutaneously in the right flank of 129/SvEv mice (females, six to eight weeks-old).

### 4.2 Biodistribution studies

The L19-IL2 immunocytokines (100 *µ*g) were radioiodinated with ¹²⁵I and Chloramine T hydrate and purified on a PD10 column. Radiolabeled immunocytokines were injected into the lateral tail vein of immunocompetent (129/Sv) mice bearing subcutaneously implanted F9 murine teratocarcinomas. The injected dose per mouse varied between 12 and 15 *µ*g. Mice were sacrificed 24 hours after injection. Organ samples were weighed and radioactivity was counted using a Packard Cobra gamma counter. The protein uptake in the different organs was calculated and expressed as the percentage of the injected dose per gram of tissue (%ID/g).

### 4.3 Immunofluorescence experiments

Immunofluorescence was performed on frozen murine F9 teratocarcinoma sections (8 *µ*m). The tumor sections were fixed using ice-cold acetone (5 min) and blocked with 20% fetal bovine serum in PBS for 45 min. The L19-IL2 immunocytokines were added to the tumour sections at a concentration of 5 *µ*g/mL in a 2% BSA/PBS solution and incubated for 1h at room temperature. Anti-human interleukin-2 (final dilution 1:150) was used as the secondary antibody to detect the L19-IL2 immunocytokines. The secondary antibody was added to the tumour sections in a 2% BSA/PBS solution and incubated at room temperature for 1h. Donkey anti-rat Alexa 488 antibody (final dilution 1:500) was used as the tertiary antibody. Nuclei were counterstained with DAPI. Slides were analyzed with an Axioskop2 mot plus microscope. All of the L19-IL2 immunocytokines showed specific binding to the vasculature compared to the negative control.

### Example 5 - Comparative biodistribution experiments between the L19 scDb N-terminal fusion protein and L19 scDb C-terminal fusion protein

### 5.1 Tumor implantation

F9 tumor cells (25 × 10⁶ cells resuspended in 200 *µ*L of HBSS buffer) were implanted subcutaneously in the right flank of 129/SvEv mice (females, six to eight weeks-old).

### 5.2 Radiolabeling and tumor targeting

The purified immunocytokines (150 *µ*g) were radioiodinated with ¹²⁵I and Chloramine T hydrate and purified on a PD10 column. Radiolabeled immunocytokines were injected into the lateral tail vein of immunocompetent (129/Sv) mice bearing subcutaneously implanted F9 murine teratocarcinoma. Injected dose per mouse varied between 14 and 17 *µ*g. Mice were sacrificed 24 hours after injection. Organ samples were weighed, and radioactivity was counted using a Packard Cobra gamma counter. The protein uptake in the different organs was calculated and expressed as the percentage of the injected dose per gram of tissue (%ID/g).

### Conclusion

Six novel immunocytokine formats comprising the VH and VL domains of the L19 antibody and an IL2 payload were generated. The L19-IL2 immunocytokines were designed to comprise different linker sequences between the VH and VL domains in diabody format (neutral, positively charged and negatively charged) (**Example 1.1**), or to have different immunocytokine formats (e.g. number of IL2 payloads per molecule) (**Examples 1.2 to 1.6**). The different L19-IL2 immunocytokines showed only minor differences when characterised *in vitro.*

Surprisingly, *in vivo* biodistribution studies performed in F9 tumour-bearing mice using radioiodinated immunocytokine preparations showed that one immunocytokine format (scDb C-terminus [L19L19IL2]) had significantly superior tumour targeting properties, as reflected by a higher percentage of the injected dose of the immunocytokine being taken up by the tumour, compared with the other immunocytokine formats tested. Specifically, the scDb C-terminal fusion protein showed an accumulation of about 7.7% ID/g in the tumour and a favourable tumor-to-organ profile, while the other immunocytokines tested only reached values of around 5% ID/g in the tumour at a maximum.

A further *in vivo* biodistribution study performed in F9 tumour-bearing mice using radioiodinated immunocytokine preparations further showed that the scDb C-terminus [L19L19IL2] immunocytokine format also had significantly superior tumour targeting properties compared with the scDb N-terminus [IL2L19L19] immunocytokine format, as well as the scFv L19-IL2 homodimer [scFv₂] immunocytokine format, as previously shown. Specifically, the scDb C-terminal fusion protein [L19L19IL2] showed an accumulation of about 7.8% ID/g in the tumour and a favourable tumor-to-organ profile, while the scDb N-terminal fusion protein [IL2L19L19] could only reach values around 3.9% ID/g and the scFv L19-IL2 homodimer around 4.6% ID/g.

Sequence listing

SEQ ID NO: 1: Interleukin-2 (IL2)




SEQ ID NO: 2: L19 VH



SEQ ID NO: 3: L19 VL



SEQ ID NO: 4: L19 VH CDR1
SFSMS

SEQ ID NO: 5: L19 VH CDR2
SISGSSGTTYYADSVKG

SEQ ID NO: 6: L19 VH CDR3
PFPYFDY

SEQ ID NO: 7: L19 VL CDR1
RASQSVSSSFLA

SEQ ID NO: 8: L19 VL CDR2
YASSRAT

SEQ ID NO: 9: L19 VL CDR3
QQTGRIPPT

SEQ ID NO: 10: L19 Diabody
L19 VH – LINKER – L19 VL

EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
SGGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIK

SEQ ID NO: 11: L19 scDb
L19 VH – LINKER – L19 VL – LINKER – L19 VH – LINKER – L19 VL

EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
SGGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKGSLDGAGGSAGA
DGGEVQLLESGGGLVQPGGSLRLSCAASGFTFSSFSMSWVRQAPGKGLEWVSSISGSS
GTTYYADSVKGRFTISRDNSKNTLYLQMNSLRAEDTAVYYCAKPFPYFDYWGQGTLVTVS
SGSSGGEIVLTQSPGTLSLSPGERATLSCRASQSVSSSFLAWYQQKPGQAPRLLIYYASSR
ATGIPDRFSGSGSGTDFTLTISRLEPEDFAVYYCQQTGRIPPTFGQGTKVEIK

SEQ ID NO: 12: L19-IL2 (scDb C-Terminus)
L19 VH – LINKER – L19 VL – LINKER – L19 VH – LINKER – L19 VL – (G4S)3 Linker –
HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
SGGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKGSLDGAGGSAGA
DGGEVQLLESGGGLVQPGGSLRLSCAASGFTFSSFSMSWVRQAPGKGLEWVSSISGSS
GTTYYADSVKGRFTISRDNSKNTLYLQMNSLRAEDTAVYYCAKPFPYFDYWGQGTLVTVS
SGSSGGEIVLTQSPGTLSLSPGERATLSCRASQSVSSSFLAWYQQKPGQAPRLLIYYASSR
ATGIPDRFSGSGSGTDFTLTISRLEPEDFAVYYCQQTGRIPPTFGQGTKVEIKGGGGSGGG
GSGGGGSAPTSSSTKKTQLQLEHLLLDLQMILNGINNYKNPKLTRMLTFKFYMPKKATELKH
LQCLEEELKPLEEVLNLAQSKNFHLRPRDLISNINVIVLELKGSETTFMCEYADETATIVEFLN
RWITFCQSIISTLT

SEQ ID NO: 13 scDb N-terminus IL2-L19

HuIL2 – (G4S)3 Linker – L19 VH – LINKER – L19 VL – Linker – L19 VH – LINKER – L19
VL

APTSSSTKKTQLQLEHLLLDLQMILNGINNYKNPKLTRMLTFKFYMPKKATELKHLQCLEEEL
KPLEEVLNLAQSKNFHLRPRDLISNINVIVLELKGSETTFMCEYADETATIVEFLNRWITFCQS
IISTLTGGGGSGGGGSGGGGSEVQLLESGGGLVQPGGSLRLSCAASGFTFSSFSMSWVR
QAPGKGLEWVSSISGSSGTTYYADSVKGRFTISRDNSKNTLYLQMNSLRAEDTAVYYCAK
PFPYFDYWGQGTLVTVSSGSSGGEIVLTQSPGTLSLSPGERATLSCRASQSVSSSFLAWY
QQKPGQAPRLLIYYASSRATGIPDRFSGSGSGTDFTLTISRLEPEDFAVYYCQQTGRIPPTF
GQGTKVEIKGSLDGAGGSAGADGGEVQLLESGGGLVQPGGSLRLSCAASGFTFSSFSMS
WVRQAPGKGLEWVSSISGSSGTTYYADSVKGRFTISRDNSKNTLYLQMNSLRAEDTAVY
YCAKPFPYFDYWGQGTLVTVSSGSSGGEIVLTQSPGTLSLSPGERATLSCRASQSVSSSFL
AWYQQKPGQAPRLLIYYASSRATGIPDRFSGSGSGTDFTLTISRLEPEDFAVYYCQQTGRIP
PTFGQGTKVEIK

SEQ ID NO: 14: L19-IL2 (Crab)
L19 VH – LINKER – L19 VL – (G4S)3 Linker – HuIL2 – (G4S)3 Linker – L19 VH – LINKER
– L19 VL

EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGG
GGSGGGGSGGGGEIVLTQSPGTLSLSPGERATLSCRASQSVSSSFLAWYQQKPGQAPRL
LIYYASSRATGIPDRFSGSGSGTDFTLTISRLEPEDFAVYYCQQTGRIPPTFGQGTKVEIKGG
GGSGGGGSGGGGSAPTSSSTKKTQLQLEHLLLDLQMILNGINNYKNPKLTRMLTFKFYMPK
KATELKHLQCLEEELKPLEEVLNLAQSKNFHLRPRDLISNINVIVLELKGSETTFMCEYADET
ATIVEFLNRWITFCQSIISTLTGGGGSGGGGSGGGGSEVQLLESGGGLVQPGGSLRLSCA
ASGFTFSSFSMSWVRQAPGKGLEWVSSISGSSGTTYYADSVKGRFTISRDNSKNTLYLQ
MNSLRAEDTAVYYCAKPFPYFDYWGQGTLVTVSSGGGGSGGGGSGGGGEIVLTQSPGT
LSLSPGERATLSCRASQSVSSSFLAWYQQKPGQAPRLLIYYASSRATGIPDRFSGSGSGTD
FTLTISRLEPEDFAVYYCQQTGRIPPTFGQGTKVEIK

SEQ ID NO: 15: L19-IL2 (scFv2)
L19 VH – LINKER – L19 VL – Linker – HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGD
GSSGGSGGASEIVLTQSPGTLSLSPGERATLSCRASQSVSSSFLAWYQQKPGQAPRLLIYY
ASSRATGIPDRFSGSGSGTDFTLTISRLEPEDFAVYYCQQTGRIPPTFGQGTKVEIKEFSSS
SGSSSSGSSSSGAPTSSSTKKTQLQLEHLLLDLQMILNGINNYKNPKLTRMLTFKFYMPKKA
TELKHLQCLEEELKPLEEVLNLAQSKNFHLRPRDLISNINVIVLELKGSETTFMCEYADETATI
VEFLNRWITFCQSIISTLT

SEQ ID NO: 16: L19-IL2 (Diabody linker 1: GSSGG)
L19 VH – LINKER – L19 VL – (S4G)3 Linker – HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
SGGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKSSSSGSSSSGSSS
SGAPTSSSTKKTQLQLEHLLLDLQMILNGINNYKNPKLTRMLTFKFYMPKKATELKHLQCLE
EELKPLEEVLNLAQSKNFHLRPRDLISNINVIVLELKGSETTFMCEYADETATIVEFLNRWITF
CQSIISTLT

SEQ ID NO: 17: L19-IL2 (Diabody linker 2: GGSGG)
L19 VH – LINKER – L19 VL – (S4G)3 Linker – HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGG
SGGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKSSSSGSSSSGSSS
SGAPTSSSTKKTQLQLEHLLLDLQMILNGINNYKNPKLTRMLTFKFYMPKKATELKHLQCLE
EELKPLEEVLNLAQSKNFHLRPRDLISNINVIVLELKGSETTFMCEYADETATIVEFLNRWITF
CQSIISTLT

SEQ ID. NO: 18: L19-IL2 (Diabody linker 3: GSADG)
L19 VH – LINKER – L19 VL – (S4G)3 Linker – HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
ADGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKSSSSGSSSSGSSS
SGAPTSSSTKKTQLQLEHLLLDLQMILNGINNYKNPKLTRMLTFKFYMPKKATELKHLQCLE
EELKPLEEVLNLAQSKNFHLRPRDLISNINVIVLELKGSETTFMCEYADETATIVEFLNRWITF
CQSIISTLT

SEQ ID NO: 19: L19-IL2 (Diabody linker 4: GSAKG)
L19 VH – LINKER – L19 VL – (S4G)3 Linker – HuIL2
EVQLLESGGGLVQPGGSLRLSCAASGFTFSSFSMSWVRQAPGKGLEWVSSISGSSGTTY
YADSVKGRFTISRDNSKNTLYLQMNSLRAEDTAVYYCAKPFPYFDYWGQGTLVTVSSGS
AKGEIVLTQSPGTLSLSPGERATLSCRASQSVSSSFLAWYQQKPGQAPRLLIYYASSRATGI
PDRFSGSGSGTDFTLTISRLEPEDFAVYYCQQTGRIPPTFGQGTKVEIKSSSSGSSSSGSSS

SGAPTSSSTKKTQLQLEHLLLDLQMILNGINNYKNPKLTRMLTFKFYMPKKATELKHLQCLE
EELKPLEEVLNLAQSKNFHLRPRDLISNINVIVLELKGSETTFMCEYADETATIVEFLNRWITF
CQSIISTLT

SEQ ID NO: 20: L19-IL2 (scDb X 2)
HuIL2– (G4S)3 Linker – L19 VH – LINKER – L19 VL – LINKER – L19 VH – LINKER – L19
VL – (G4S)3 Linker – HuIL2
APTSSSTKKTQLQLEHLLLDLQMILNGINNYKNPKLTRMLTFKFYMPKKATELKHLQCLEEEL
KPLEEVLNLAQSKNFHLRPRDLISNINVIVLELKGSETTFMCEYADETATIVEFLNRWITFCQS
IISTLTGGGGSGGGGSGGGGSEVQLLESGGGLVQPGGSLRLSCAASGFTFSSFSMSWVR
QAPGKGLEWVSSISGSSGTTYYADSVKGRFTISRDNSKNTLYLQMNSLRAEDTAVYYCAK
PFPYFDYWGQGTLVTVSSGSSGGEIVLTQSPGTLSLSPGERATLSCRASQSVSSSFLAWY
QQKPGQAPRLLIYYASSRATGIPDRFSGSGSGTDFTLTISRLEPEDFAVYYCQQTGRIPPTF
GQGTKVEIKGSLDGAGGSAGADGGEVQLLESGGGLVQPGGSLRLSCAASGFTFSSFSMS
WVRQAPGKGLEWVSSISGSSGTTYYADSVKGRFTISRDNSKNTLYLQMNSLRAEDTAVY
YCAKPFPYFDYWGQGTLVTVSSGSSGGEIVLTQSPGTLSLSPGERATLSCRASQSVSSSFL
AWYQQKPGQAPRLLIYYASSRATGIPDRFSGSGSGTDFTLTISRLEPEDFAVYYCQQTGRIP
PTFGQGTKVEIKGGGGSGGGGSGGGGSAPTSSSTKKTQLQLEHLLLDLQMILNGINNYKNP
KLTRMLTFKFYMPKKATELKHLQCLEEELKPLEEVLNLAQSKNFHLRPRDLISNINVIVLELKG
SETTFMCEYADETATIVEFLNRWITFCQSIISTLT

SEQ ID NO: 21: Linker between VH and VL in diabody and scDb
GSSGG

SEQ ID NO: 22: Linker between VH and VL in diabody
GGSGG

SEQ ID NO: 23: Linker between VH and VL in diabody
GSADG

SEQ ID NO: 24: Linker between VH and VL in diabody
GSAKG

SEQ ID NO: 25: Linker between Diabody in scDb
GSLDGAGGSAGADGG

SEQ ID NO: 26: Linker between VL or VH and IL2

GGGGSGGGGSGGGGS

SEQ ID NO: 27 Clones GGSGG, GSADG, and GSAKG Fragment A Forward Primer
TAATACGACTCACTATAGGG

SEQ ID NO: 28 Clone GGSGG Fragment A Backward Primer
CACCGCCTGATCCCCCACTCGAGACGGTGACCAGGGT

SEQ ID NO: 29 Clone GSADG Fragment A Backward Primer
CGTCTGCTGACCCACTCGAGACGGTGACCAGGGTTCCC

SEQ ID NO: 30 Clone GSAKG Fragment A Backward Primer
ACCTTTTGCTGACCCACTCGAGACGGTGACCAGGGTTCCC

SEQ ID NO: 31 Clone GGSGG Fragment B Forward Primer
TCTCGAGTGGGGGATCAGGCGGTGAAATTGTGTTGACGCAG

SEQ ID NO: 32 Clone GGSGG, GSADG, and GSAKG Fragment B Backward Primer
TAGAAGGCACAGTCGAGG

SEQ ID NO: 33 Clone GSADG Fragment B Forward Primer
CCGTCTCGAGTGGGTCAGCAGACGGTGAAATTGTGTTGACGCAGTCT

SEQ ID NO: 34 Clone GSAKG Fragment B Forward Primer
CCGTCTCGAGTGGGTCAGCAAAAGGTGAAATTGTGTTGACGCAGTCT

SEQ ID NO: 35 Primer NheLead>
CTAGCTAGCTAGGACCATGGGCTGGAGCCTGATCCTCCTGTTCCTCGTCGCTGTGGCT
A

SEQ ID NO: 36 Primer IL2G4S3<
CCGCCAGAACCCCCTCCGCCTGACCCGCCTCCACCAGTCAGTGTTGAGATGATGCTTT
G

SEQ ID NO: 37 Primer G4S3L19>
GGTCAGGCGGAGGGGGTTCTGGCGGTGGCGGATCGGAGGTGCAGCTGTTGGAGTCT
GGG

SEQ ID NO: 38 Primer L19Hind<
GAAGCTTCCTTTGATTTCCACCTTGGTCCCTTG

SEQ ID NO: 39 Primer LnkDP47>
ATGGAGCAGGTGGCAGTGCAGGAGCGGACGGGGGTGAGGTGCAGCTGTTGGAGTCT
GGG

SEQ ID NO: 40 Primer HindLnk>
CAAGCTTGGATGGAGCAGGTGGCAGTGCAGGAG

SEQ ID NO: 41 Primer G4S3IL2>
GGTCAGGCGGAGGGGGTTCTGGCGGTGGCGGATCGGCACCTACTTCAAGTTCTACAA
AG

SEQ ID NO: 42 Primer L19G4S3<
CCGCCAGAACCCCCTCCGCCTGACCCGCCTCCACCTTTGATTTCCACCTTGGTCCCTT
G

SEQ ID NO: 43 Primer IL2G4S3Bam<
CGCGGATCCCCCTCCGCCTGACCCGCCTCCACCAGTCAGTGTTGAGATGATGCTTTG

SEQ ID NO: 44 Primer BamG4S3L19>
CGCGGATCCGGCGGTGGCGGATCGGAGGTGCAGCTGTTGGAGTCTGGG

SEQ ID NO: 45 Primer L19StopNot<
TTTTCCTTTTGCGGCCGCTCATTATTTGATTTCCACCTTGGTCCCTTG

SEQ ID NO: 46 Primer IL2StopNot<
TTTTCCTTTTGCGGCCGCTCATTAAGTCAGTGTTGAGATGATGCTTTG

SEQ ID NO: 47 Primer DP47G4S2.5<
CCCTGACCCTCCGCCACCAGAGCCCCCACCTCCACTCGAGACGGTGACCAGGGTTCC

SEQ ID NO: 48 Primer G4S2.5VL>
GGGCTCTGGTGGCGGAGGGTCAGGGGGAGGCGGTGAAATTGTGTTGACGCAGTCTCC
A

SEQ ID NO: 49 Primer LeadIL2>
CTGTTCCTCGTCGCTGTGGCTACAGGTGTGCACTCGGCACCTACTTCAAGTTCTACAAA

SEQ ID NO: 50 Primer HindIIISIP
CCCAAGCTTGTCGACCATGGGCTGGAGCC

SEQ ID NO: 51 Primer L19Linker
GAGCCGGAAGAGCTACTACCCGATGAGGAAGATTTGATTTCCACCTTGGTCCCTTG

SEQ ID NO: 52 Primer LinkerIL2
TCGGGTAGTAGCTCTTCCGGCTCATCGTCCAGCGGCGCACCTACTTCAAGTTCTACA

SEQ ID NO: 53 Primer IL2stopNotI
TTTTCCTTTTGCGGCCGCTCATTAAGTCAGTGTTGAGATGAT

SEQ ID NO: 54: F8 VH
EVQLLESGGGLVQPGGSLRLSCAASGFTFSLFTMSWVRQAPGKGLEWVSAISGSGGSTYY
ADSVKGRFTISRDNSKNTLYLQMNSLRAEDTAVYYCAKSTHLYLFDYWGQGTLVTVSS

SEQ ID NO: 55: F8 VL
EIVLTQSPGTLSLSPGERATLSCRASQSVSMPFLAWYQQKPGQAPRLLIYGASSRATGIPD
RFSGSGSGTDFTLTISRLEPEDFAVYYCQQMRGRPPTFGQGTKVEIK

SEQ ID NO: 56: F8 VH CDR1
LFT

SEQ ID NO: 57: F8 VH CDR2
SGSGGS

SEQ ID NO: 58: F8 VH CDR3
STHLYL

SEQ ID NO: 59: F8 VL CDR1
MPF

SEQ ID NO: 60: F8 VL CDR2

GASSRAT

SEQ ID NO: 61: F8 VL CDR3
MRGRPP

SEQ ID NO: 62: F8 scDb
F8 VH – LINKER – F8 VL – LINKER – F8 VH – LINKER – F8 VL

EVQLLESGGGLVQPGGSLRLSCAASGFTFSLFTMSWVRQAPGKGLEWVSAISGSGGSTY
YADSVKGRFTISRDNSKNTLYLQMNSLRAEDTAVYYCAKSTHLYLFDYWGQGTLVTVSSG
SSGGEIVLTQSPGTLSLSPGERATLSCRASQSVSMPFLAWYQQKPGQAPRLLIYGASSRAT
GIPDRFSGSGSGTDFTLTISRLEPEDFAVYYCQQMRGRPPTFGQGTKVEIKGSLDGAGGS
AGADGGEVQLLESGGGLVQPGGSLRLSCAASGFTFSLFTMSWVRQAPGKGLEWVSAIS
GSGGSTYYADSVKGRFTISRDNSKNTLYLQMNSLRAEDTAVYYCAKSTHLYLFDYWGQG
TLVTVSSGSSGGEIVLTQSPGTLSLSPGERATLSCRASQSVSMPFLAWYQQKPGQAPRLLI
YGASSRATGIPDRFSGSGSGTDFTLTISRLEPEDFAVYYCQQMRGRPPTFGQGTKVEIK

SEQ ID NO: 63: F16 VH
EVQLLESGGGLVQPGGSLRLSCAASGFTFSRYGMSWVRQAPGKGLEWVSAISGSGGSTY
YADSVKGRFTISRDNSKNTLYLQMNSLRAEDTAVYYCAKAHNAFDYWGQGTLVTVSR

SEQ ID NO: 64: F16 VL
SSELTQDPAVSVALGQTVRITCQGDSLRSYYASWYQQKPGQAPVLVIYGKNNRPSGIPDRF
SGSSSGNTASLTITGAQAEDEADYYCNSSVYTMPPVVFGGGTKLTVLG

SEQ ID NO: 65: F16 VH CDR1
RYGMS

SEQ ID NO: 66: F16 VH CDR2
AISGSGGSTYYADSVKG

SEQ ID NO: 67: F16 VH CDR3
AHNAFDY

SEQ ID NO: 68: F16 VL CDR1
QGDSLRSYYAS

SEQ ID NO: 69: F16 VL CDR2
GKNNRPS

SEQ ID NO: 70: F16 VL CDR3
NSSVYTMPPVV

SEQ ID NO: 71: F16 scDb
F16 VH – LINKER – F16 VL – LINKER – F16 VH – LINKER – F16 VL

EVQLLESGGGLVQPGGSLRLSCAASGFTFSRYGMSWVRQAPGKGLEWVSAISGSGGST
YYADSVKGRFTISRDNSKNTLYLQMNSLRAEDTAVYYCAKAHNAFDYWGQGTLVTVSRG
SSGGSSELTQDPAVSVALGQTVRITCQGDSLRSYYASWYQQKPGQAPVLVIYGKNNRPSG
IPDRFSGSSSGNTASLTITGAQAEDEADYYCNSSVYTMPPVVFGGGTKLTVLGGSLDGAG
GSAGADGGEVQLLESGGGLVQPGGSLRLSCAASGFTFSRYGMSWVRQAPGKGLEWVS
AISGSGGSTYYADSVKGRFTISRDNSKNTLYLQMNSLRAEDTAVYYCAKAHNAFDYWGQ
GTLVTVSRGSSGGSSELTQDPAVSVALGQTVRITCQGDSLRSYYASWYQQKPGQAPVLVI
YGKNNRPSGIPDRFSGSSSGNTASLTITGAQAEDEADYYCNSSVYTMPPVVFGGGTKLTVL
G

### References

All documents mentioned in this specification are incorporated herein by reference in their entirety.
Borsi et al. (1987), J. Cell. Biol., 104, 595-600
Borsi et al. (2002) Int. J. Cancer: 102, 75-85
Brack, S.S., Silacci, M., Birchler, M. & Neri, D. Tumor-targeting properties of novel antibodies specific to the large isoform of tenascin-C. Clin Cancer Res 12, 3200-3208 (2006).
Dela Cruz, J.S., Huang, T.H., Penichet, M.L. & Morrison, S.L. Antibody-cytokine fusion proteins: innovative weapons in the war against cancer. Clin Exp Med 4, 57-64 (2004).
Folkman, J. Angiogenesis in cancer, vascular, rheumatoid and other disease. Nat Med 1, 27-31 (1995).
Holliger and Winter. Diabodies: small bispecific antibody fragments. Cancer Immunol Immunother (1997) 45: 128-130.
Holliger et al., Proc. Natl. Acad. Sci. USA 90 6444-6448, 1993.
Jain, R.K. & Baxter, L.T. Mechanisms of heterogeneous distribution of monoclonal antibodies and other macromolecules in tumors: significance of elevated interstitial pressure. Cancer Res 48, 7022-7032 (1988).
Johannsen, M., et al. The tumour-targeting human L19-IL2 immunocytokine: preclinical safety studies, phase I clinical trial in patients with solid tumours and expansion into patients with advanced renal cell carcinoma. Eur J Cancer 46, 2926-2935 (2010).
Kontermann, R. E., and Muller, R. (1999). Intracellular and cell surface display of single-chain diabodies. J. Immunol. Methods 226: 179-188.
Neri, D. & Bicknell, R. Tumour vascular targeting. Nat Rev Cancer 5, 436-446 (2005).
Nettelbeck, D.M; Miller, D.W; Jérôme, V; Zuzarte, M; Watkins, S. J; Hawkins, R.E; Muller, R; and Kontermann, R.E. Molecular Therapy (2001) 3, 882-891.
Pini, A., et al. Design and use of a phage display library. Human antibodies with subnanomolar affinity against a marker of angiogenesis eluted from a two-dimensional gel. J Biol Chem 273, 21769-21776 (1998).
Reisfeld, R.A., Becker, J.C. & Gillies, S.D. Immunocytokines: a new approach to immunotherapy of melanoma. Melanoma Res 7 Suppl 2, S99-106 (1997).
Sauer, S., et al. Expression of the oncofetal ED-B containing fibronectin isoform in hematologic tumors enables ED-B targeted 131I-L19SIP radioimmunotherapy in Hodgkin lymphoma patients. Blood (2009).
Savage, P., So, A., Spooner, R.A. & Epenetos, A.A. A recombinant single chain antibody interleukin-2 fusion protein. Br J Cancer 67, 304-310 (1993).
Schrama, D., Reisfeld, R.A. & Becker, J.C. Antibody targeted drugs as cancer therapeutics. Nat Rev Drug Discov 5, 147-159 (2006).
Villa, A., et al. A high-affinity human monoclonal antibody specific to the alternatively spliced EDA domain of fibronectin efficiently targets tumor neo-vasculature in vivo. Int J Cancer 122, 2405-2413 (2008).
Viti et al. (1999) Cancer Res. 59(2): 347-52.

## Claims

1. A conjugate comprising interleukin-2 (IL2) and a single-chain diabody, wherein the single-chain diabody binds an extracellular matrix component associated with neoplastic growth and/or angiogenesis, wherein the IL2 is linked to the C-terminus or the N-terminus of the single-chain diabody.

2. The conjugate according to claim 1, wherein the IL2 is linked to the C-terminus of the single-chain diabody and the single-chain diabody has a free N-terminus, or wherein the IL2 is linked to the N-terminus of the single-chain diabody and the single-chain diabody has a free C-terminus.

3. The conjugate according to any one of the preceding claims, wherein the extracellular matrix component associated with neoplastic growth and/or angiogenesis is fibronectin.

4. The conjugate according to claim 3, wherein the fibronectin is the Extra Domain-B (ED-B) of fibronectin.

5. The conjugate according to any one of the preceding claims, wherein the single-chain diabody comprises an antigen-binding site having the complementarity determining regions (CDRs) of antibody L19 set forth in SEQ ID NOs 4 to 9.

6. The conjugate according to claim 5, wherein the single-chain diabody comprises the VH and VL domains of antibody L19 set forth in SEQ ID NOs 2 and 3.

7. The conjugate according to claim 6, wherein the single-chain diabody comprises the amino acid sequence set forth in SEQ ID NO: 11.

8. The conjugate according to any one of the preceding claims, wherein the IL2 is human IL2.

9. The conjugate according to claim 9, wherein the IL2 comprises the sequence set forth in SEQ ID NO: 1.

10. The conjugate according to claim 9, wherein the conjugate comprises the sequence set forth in SEQ ID NO: 12 or 13.

11. The conjugate according to any one of the preceding claims which is a single-chain fusion protein.

12. A nucleic acid molecule encoding a conjugate according to any one of claims 1 to 11.

13. An expression vector comprising the nucleic acid of claim 12.

14. A host cell comprising the nucleic acid molecule of claim 12 or vector of claim 13.

15. A method of producing a conjugate according to any one of claims 1 to 11, the method comprising culturing the host cell of claim 14 under conditions for expression of the conjugate.

16. The method of claim 15 further comprising isolating and/or purifying the conjugate.

17. The conjugate according to any of claims 1 to 11 for use in a method of treating cancer in a patient by targeting IL2 to the neovasculature *in vivo.*

18. The conjugate according to any one of claims 1 to 11 for use in a method of delivering IL2 to the tumour neovasculature in a patient.

19. A method of treating cancer by targeting IL2 to the neovasculature in a patient, the method comprising administering a therapeutically effective amount of a conjugate according to any of claims 1 to 11 to the patient.

20. A method of delivering IL2 to the tumour neovasculature in a patient comprising administering to the patient a conjugate according to any one of claims 1 to 11.


SEQUENCE LISTING

<110>  Philogen S.P.A.

<120>  IL2 Immunoconjugates

<130>  TEK/CP7430598

<140>  EP 18209671.9
<141>  2018-11-30

<160>  71

<170>  PatentIn version 3.5

<210>  1
<211>  133
<212>  PRT
<213>  Artificial Sequence

<220>
<223>  Interleukin-2 (IL2)

<400>  1

<210>  2
<211>  116

<212>  PRT
<213>  Artificial Sequence

<220>
<223>  L19 VH

<400>  2


<210>  3
<211>  108
<212>  PRT
<213>  Artificial Sequence

<220>
<223>  L19 VL

<400>  3

<210>  4
<211>  5
<212>  PRT
<213>  Artificial Sequence

<220>
<223>  L19 VH CDR1

<400>  4

Ser Phe Ser Met Ser
1               5

<210>  5
<211>  17
<212>  PRT
<213>  Artificial Sequence

<220>
<223>  L19 VH CDR2

<400>  5

Ser Ile Ser Gly Ser Ser Gly Thr Thr Tyr Tyr Ala Asp Ser Val Lys
1               5                   10                  15

Gly

<210>  6
<211>  7
<212>  PRT
<213>  Artificial Sequence

<220>
<223>  L19 VH CDR3

<400>  6

Pro Phe Pro Tyr Phe Asp Tyr
1               5

<210>  7
<211>  12

<212>  PRT
<213>  Artificial Sequence

<220>
<223>  L19 VL CDR1

<400>  7

Arg Ala Ser Gln Ser Val Ser Ser Ser Phe Leu Ala
1               5                   10


<210>  8
<211>  7
<212>  PRT
<213>  Artificial Sequence

<220>
<223>  L19 VL CDR2

<400>  8

Tyr Ala Ser Ser Arg Ala Thr
1               5


<210>  9
<211>  9
<212>  PRT
<213>  Artificial Sequence

<220>
<223>  L19 VL CDR3

<400>  9

Gln Gln Thr Gly Arg Ile Pro Pro Thr
1               5


<210>  10
<211>  229
<212>  PRT
<213>  Artificial Sequence

<220>
<223>  L19 Diabody

<400>  10

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Phe
            20                  25                  30


Ser Met Ser Trp Val Arg Gln Ala Pro Gly Lys Gly Leu Glu Trp Val
        35                  40                  45


Ser Ser Ile Ser Gly Ser Ser Gly Thr Thr Tyr Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ser Gly Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys
225

<210>  11
<211>  473
<212>  PRT
<213>  Artificial Sequence

<220>
<223>  L19 scDb

<400>  11

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ser Gly Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Gly Ser Leu Asp Gly Ala Gly Gly Ser Ala Gly
225                 230                 235                 240

Ala Asp Gly Gly Glu Val Gln Leu Leu Glu Ser Gly Gly Gly Leu Val
                245                 250                 255

Gln Pro Gly Gly Ser Leu Arg Leu Ser Cys Ala Ala Ser Gly Phe Thr
            260                 265                 270

Phe Ser Ser Phe Ser Met Ser Trp Val Arg Gln Ala Pro Gly Lys Gly

275                 280                 285

Leu Glu Trp Val Ser Ser Ile Ser Gly Ser Ser Gly Thr Thr Tyr Tyr
    290                 295                 300

Ala Asp Ser Val Lys Gly Arg Phe Thr Ile Ser Arg Asp Asn Ser Lys
305                 310                 315                 320

Asn Thr Leu Tyr Leu Gln Met Asn Ser Leu Arg Ala Glu Asp Thr Ala
                325                 330                 335

Val Tyr Tyr Cys Ala Lys Pro Phe Pro Tyr Phe Asp Tyr Trp Gly Gln
            340                 345                 350

Gly Thr Leu Val Thr Val Ser Ser Gly Ser Ser Gly Gly Glu Ile Val
        355                 360                 365

Leu Thr Gln Ser Pro Gly Thr Leu Ser Leu Ser Pro Gly Glu Arg Ala
    370                 375                 380

Thr Leu Ser Cys Arg Ala Ser Gln Ser Val Ser Ser Ser Phe Leu Ala
385                 390                 395                 400

Trp Tyr Gln Gln Lys Pro Gly Gln Ala Pro Arg Leu Leu Ile Tyr Tyr
                405                 410                 415

Ala Ser Ser Arg Ala Thr Gly Ile Pro Asp Arg Phe Ser Gly Ser Gly
            420                 425                 430

Ser Gly Thr Asp Phe Thr Leu Thr Ile Ser Arg Leu Glu Pro Glu Asp
        435                 440                 445

Phe Ala Val Tyr Tyr Cys Gln Gln Thr Gly Arg Ile Pro Pro Thr Phe
    450                 455                 460

Gly Gln Gly Thr Lys Val Glu Ile Lys
465                 470

<210>  12
<211>  621
<212>  PRT
<213>  Artificial Sequence

<220>
<223>  L19-IL2 (scDb C-Terminus)

<400>  12

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ser Gly Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Gly Ser Leu Asp Gly Ala Gly Gly Ser Ala Gly
225                 230                 235                 240

Ala Asp Gly Gly Glu Val Gln Leu Leu Glu Ser Gly Gly Gly Leu Val
                245                 250                 255

Gln Pro Gly Gly Ser Leu Arg Leu Ser Cys Ala Ala Ser Gly Phe Thr

260                 265                 270

Phe Ser Ser Phe Ser Met Ser Trp Val Arg Gln Ala Pro Gly Lys Gly
        275                 280                 285

Leu Glu Trp Val Ser Ser Ile Ser Gly Ser Ser Gly Thr Thr Tyr Tyr
    290                 295                 300

Ala Asp Ser Val Lys Gly Arg Phe Thr Ile Ser Arg Asp Asn Ser Lys
305                 310                 315                 320

Asn Thr Leu Tyr Leu Gln Met Asn Ser Leu Arg Ala Glu Asp Thr Ala
                325                 330                 335

Val Tyr Tyr Cys Ala Lys Pro Phe Pro Tyr Phe Asp Tyr Trp Gly Gln
            340                 345                 350

Gly Thr Leu Val Thr Val Ser Ser Gly Ser Ser Gly Gly Glu Ile Val
        355                 360                 365

Leu Thr Gln Ser Pro Gly Thr Leu Ser Leu Ser Pro Gly Glu Arg Ala
    370                 375                 380

Thr Leu Ser Cys Arg Ala Ser Gln Ser Val Ser Ser Ser Phe Leu Ala
385                 390                 395                 400

Trp Tyr Gln Gln Lys Pro Gly Gln Ala Pro Arg Leu Leu Ile Tyr Tyr
                405                 410                 415

Ala Ser Ser Arg Ala Thr Gly Ile Pro Asp Arg Phe Ser Gly Ser Gly
            420                 425                 430

Ser Gly Thr Asp Phe Thr Leu Thr Ile Ser Arg Leu Glu Pro Glu Asp
        435                 440                 445

Phe Ala Val Tyr Tyr Cys Gln Gln Thr Gly Arg Ile Pro Pro Thr Phe
    450                 455                 460

Gly Gln Gly Thr Lys Val Glu Ile Lys Gly Gly Gly Gly Ser Gly Gly
465                 470                 475                 480

Gly Gly Ser Gly Gly Gly Gly Ser Ala Pro Thr Ser Ser Ser Thr Lys
                485                 490                 495

Lys Thr Gln Leu Gln Leu Glu His Leu Leu Leu Asp Leu Gln Met Ile
            500                 505                 510

Leu Asn Gly Ile Asn Asn Tyr Lys Asn Pro Lys Leu Thr Arg Met Leu
        515                 520                 525

Thr Phe Lys Phe Tyr Met Pro Lys Lys Ala Thr Glu Leu Lys His Leu
    530                 535                 540

Gln Cys Leu Glu Glu Glu Leu Lys Pro Leu Glu Glu Val Leu Asn Leu
545                 550                 555                 560

Ala Gln Ser Lys Asn Phe His Leu Arg Pro Arg Asp Leu Ile Ser Asn
                565                 570                 575

Ile Asn Val Ile Val Leu Glu Leu Lys Gly Ser Glu Thr Thr Phe Met
            580                 585                 590

Cys Glu Tyr Ala Asp Glu Thr Ala Thr Ile Val Glu Phe Leu Asn Arg
        595                 600                 605

Trp Ile Thr Phe Cys Gln Ser Ile Ile Ser Thr Leu Thr
    610                 615                 620

<210>  13
<211>  621
<212>  PRT
<213>  Artificial Sequence

<220>
<223>  scDb N-terminus IL2-L19

<400>  13

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala

            100                 105                 110

Thr Ile Val Glu Phe Leu Asn Arg Trp Ile Thr Phe Cys Gln Ser Ile
        115                 120                 125

Ile Ser Thr Leu Thr Gly Gly Gly Gly Ser Gly Gly Gly Gly Ser Gly
    130                 135                 140

Gly Gly Gly Ser Glu Val Gln Leu Leu Glu Ser Gly Gly Gly Leu Val
145                 150                 155                 160

Gln Pro Gly Gly Ser Leu Arg Leu Ser Cys Ala Ala Ser Gly Phe Thr
                165                 170                 175

Phe Ser Ser Phe Ser Met Ser Trp Val Arg Gln Ala Pro Gly Lys Gly
            180                 185                 190

Leu Glu Trp Val Ser Ser Ile Ser Gly Ser Ser Gly Thr Thr Tyr Tyr
        195                 200                 205

Ala Asp Ser Val Lys Gly Arg Phe Thr Ile Ser Arg Asp Asn Ser Lys
    210                 215                 220

Asn Thr Leu Tyr Leu Gln Met Asn Ser Leu Arg Ala Glu Asp Thr Ala
225                 230                 235                 240

Val Tyr Tyr Cys Ala Lys Pro Phe Pro Tyr Phe Asp Tyr Trp Gly Gln
                245                 250                 255

Gly Thr Leu Val Thr Val Ser Ser Gly Ser Ser Gly Gly Glu Ile Val
            260                 265                 270

Leu Thr Gln Ser Pro Gly Thr Leu Ser Leu Ser Pro Gly Glu Arg Ala
        275                 280                 285

Thr Leu Ser Cys Arg Ala Ser Gln Ser Val Ser Ser Ser Phe Leu Ala
    290                 295                 300

Trp Tyr Gln Gln Lys Pro Gly Gln Ala Pro Arg Leu Leu Ile Tyr Tyr
305                 310                 315                 320

Ala Ser Ser Arg Ala Thr Gly Ile Pro Asp Arg Phe Ser Gly Ser Gly
                325                 330                 335

Ser Gly Thr Asp Phe Thr Leu Thr Ile Ser Arg Leu Glu Pro Glu Asp
            340                 345                 350

Phe Ala Val Tyr Tyr Cys Gln Gln Thr Gly Arg Ile Pro Pro Thr Phe
        355                 360                 365

Gly Gln Gly Thr Lys Val Glu Ile Lys Gly Ser Leu Asp Gly Ala Gly
    370                 375                 380

Gly Ser Ala Gly Ala Asp Gly Gly Glu Val Gln Leu Leu Glu Ser Gly
385                 390                 395                 400

Gly Gly Leu Val Gln Pro Gly Gly Ser Leu Arg Leu Ser Cys Ala Ala
                405                 410                 415

Ser Gly Phe Thr Phe Ser Ser Phe Ser Met Ser Trp Val Arg Gln Ala
            420                 425                 430

Pro Gly Lys Gly Leu Glu Trp Val Ser Ser Ile Ser Gly Ser Ser Gly
        435                 440                 445

Thr Thr Tyr Tyr Ala Asp Ser Val Lys Gly Arg Phe Thr Ile Ser Arg
    450                 455                 460

Asp Asn Ser Lys Asn Thr Leu Tyr Leu Gln Met Asn Ser Leu Arg Ala
465                 470                 475                 480

Glu Asp Thr Ala Val Tyr Tyr Cys Ala Lys Pro Phe Pro Tyr Phe Asp
                485                 490                 495

Tyr Trp Gly Gln Gly Thr Leu Val Thr Val Ser Ser Gly Ser Ser Gly
            500                 505                 510

Gly Glu Ile Val Leu Thr Gln Ser Pro Gly Thr Leu Ser Leu Ser Pro
        515                 520                 525

Gly Glu Arg Ala Thr Leu Ser Cys Arg Ala Ser Gln Ser Val Ser Ser
    530                 535                 540

Ser Phe Leu Ala Trp Tyr Gln Gln Lys Pro Gly Gln Ala Pro Arg Leu
545                 550                 555                 560

Leu Ile Tyr Tyr Ala Ser Ser Arg Ala Thr Gly Ile Pro Asp Arg Phe
                565                 570                 575

Ser Gly Ser Gly Ser Gly Thr Asp Phe Thr Leu Thr Ile Ser Arg Leu
            580                 585                 590

Glu Pro Glu Asp Phe Ala Val Tyr Tyr Cys Gln Gln Thr Gly Arg Ile
        595                 600                 605

Pro Pro Thr Phe Gly Gln Gly Thr Lys Val Glu Ile Lys
    610                 615                 620

<210>  14
<211>  639
<212>  PRT
<213>  Artificial Sequence

<220>
<223>  L19-IL2 (Crab)

<400>  14

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Gly Ser Gly Gly Gly Gly Ser Gly Gly Gly Gly Ser Ala Pro Thr
                245                 250                 255

Ser Ser Ser Thr Lys Lys Thr Gln Leu Gln Leu Glu His Leu Leu Leu
            260                 265                 270

Asp Leu Gln Met Ile Leu Asn Gly Ile Asn Asn Tyr Lys Asn Pro Lys
        275                 280                 285

Leu Thr Arg Met Leu Thr Phe Lys Phe Tyr Met Pro Lys Lys Ala Thr
    290                 295                 300

Glu Leu Lys His Leu Gln Cys Leu Glu Glu Glu Leu Lys Pro Leu Glu
305                 310                 315                 320

Glu Val Leu Asn Leu Ala Gln Ser Lys Asn Phe His Leu Arg Pro Arg
                325                 330                 335

Asp Leu Ile Ser Asn Ile Asn Val Ile Val Leu Glu Leu Lys Gly Ser
            340                 345                 350

Glu Thr Thr Phe Met Cys Glu Tyr Ala Asp Glu Thr Ala Thr Ile Val
        355                 360                 365

Glu Phe Leu Asn Arg Trp Ile Thr Phe Cys Gln Ser Ile Ile Ser Thr
    370                 375                 380

Leu Thr Gly Gly Gly Gly Ser Gly Gly Gly Gly Ser Gly Gly Gly Gly
385                 390                 395                 400

Ser Glu Val Gln Leu Leu Glu Ser Gly Gly Gly Leu Val Gln Pro Gly
                405                 410                 415

Gly Ser Leu Arg Leu Ser Cys Ala Ala Ser Gly Phe Thr Phe Ser Ser
            420                 425                 430

Phe Ser Met Ser Trp Val Arg Gln Ala Pro Gly Lys Gly Leu Glu Trp
        435                 440                 445

Val Ser Ser Ile Ser Gly Ser Ser Gly Thr Thr Tyr Tyr Ala Asp Ser
    450                 455                 460

Val Lys Gly Arg Phe Thr Ile Ser Arg Asp Asn Ser Lys Asn Thr Leu
465                 470                 475                 480

Tyr Leu Gln Met Asn Ser Leu Arg Ala Glu Asp Thr Ala Val Tyr Tyr
                485                 490                 495

Cys Ala Lys Pro Phe Pro Tyr Phe Asp Tyr Trp Gly Gln Gly Thr Leu
            500                 505                 510

Val Thr Val Ser Ser Gly Gly Gly Gly Ser Gly Gly Gly Gly Ser Gly
        515                 520                 525

Gly Gly Gly Glu Ile Val Leu Thr Gln Ser Pro Gly Thr Leu Ser Leu
    530                 535                 540

Ser Pro Gly Glu Arg Ala Thr Leu Ser Cys Arg Ala Ser Gln Ser Val
545                 550                 555                 560

Ser Ser Ser Phe Leu Ala Trp Tyr Gln Gln Lys Pro Gly Gln Ala Pro
                565                 570                 575

Arg Leu Leu Ile Tyr Tyr Ala Ser Ser Arg Ala Thr Gly Ile Pro Asp
            580                 585                 590

Arg Phe Ser Gly Ser Gly Ser Gly Thr Asp Phe Thr Leu Thr Ile Ser
        595                 600                 605

Arg Leu Glu Pro Glu Asp Phe Ala Val Tyr Tyr Cys Gln Gln Thr Gly
    610                 615                 620

Arg Ile Pro Pro Thr Phe Gly Gln Gly Thr Lys Val Glu Ile Lys
625                 630                 635

<210>  15
<211>  386
<212>  PRT
<213>  Artificial Sequence

<220>
<223>  L19-IL2 (scFv2)

<400>  15

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Glu Phe Ser Ser
225                 230                 235                 240

Ser Ser Gly Ser Ser Ser Ser Gly Ser Ser Ser Ser Gly Ala Pro Thr
                245                 250                 255

Ser Ser Ser Thr Lys Lys Thr Gln Leu Gln Leu Glu His Leu Leu Leu
            260                 265                 270

Asp Leu Gln Met Ile Leu Asn Gly Ile Asn Asn Tyr Lys Asn Pro Lys
        275                 280                 285

Leu Thr Arg Met Leu Thr Phe Lys Phe Tyr Met Pro Lys Lys Ala Thr
    290                 295                 300

Glu Leu Lys His Leu Gln Cys Leu Glu Glu Glu Leu Lys Pro Leu Glu
305                 310                 315                 320

Glu Val Leu Asn Leu Ala Gln Ser Lys Asn Phe His Leu Arg Pro Arg
                325                 330                 335

Asp Leu Ile Ser Asn Ile Asn Val Ile Val Leu Glu Leu Lys Gly Ser
            340                 345                 350

Glu Thr Thr Phe Met Cys Glu Tyr Ala Asp Glu Thr Ala Thr Ile Val
        355                 360                 365

Glu Phe Leu Asn Arg Trp Ile Thr Phe Cys Gln Ser Ile Ile Ser Thr
    370                 375                 380

Leu Thr
385

<210>  16
<211>  377
<212>  PRT
<213>  Artificial Sequence

<220>
<223>  L19-IL2 (Diabody linker 1: GSSGG)

<400>  16

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ser Gly Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Ser Ser Ser Ser Gly Ser Ser Ser Ser Gly Ser
225                 230                 235                 240

Ser Ser Ser Gly Ala Pro Thr Ser Ser Ser Thr Lys Lys Thr Gln Leu
                245                 250                 255

Gln Leu Glu His Leu Leu Leu Asp Leu Gln Met Ile Leu Asn Gly Ile
            260                 265                 270

Asn Asn Tyr Lys Asn Pro Lys Leu Thr Arg Met Leu Thr Phe Lys Phe
        275                 280                 285

Tyr Met Pro Lys Lys Ala Thr Glu Leu Lys His Leu Gln Cys Leu Glu
    290                 295                 300

Glu Glu Leu Lys Pro Leu Glu Glu Val Leu Asn Leu Ala Gln Ser Lys
305                 310                 315                 320

Asn Phe His Leu Arg Pro Arg Asp Leu Ile Ser Asn Ile Asn Val Ile
                325                 330                 335

Val Leu Glu Leu Lys Gly Ser Glu Thr Thr Phe Met Cys Glu Tyr Ala
            340                 345                 350

Asp Glu Thr Ala Thr Ile Val Glu Phe Leu Asn Arg Trp Ile Thr Phe
        355                 360                 365

Cys Gln Ser Ile Ile Ser Thr Leu Thr
    370                 375

<210> 17
<211> 377
<212> PRT
<213> Artificial Sequence

<220>
<223> L19-IL2 (Diabody linker 2: GGSGG)

<400> 17

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Gly Ser Gly Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Ser Ser Ser Ser Gly Ser Ser Ser Ser Gly Ser
225                 230                 235                 240

Ser Ser Ser Gly Ala Pro Thr Ser Ser Ser Thr Lys Lys Thr Gln Leu
                245                 250                 255

Gln Leu Glu His Leu Leu Leu Asp Leu Gln Met Ile Leu Asn Gly Ile
            260                 265                 270

Asn Asn Tyr Lys Asn Pro Lys Leu Thr Arg Met Leu Thr Phe Lys Phe
        275                 280                 285

Tyr Met Pro Lys Lys Ala Thr Glu Leu Lys His Leu Gln Cys Leu Glu
    290                 295                 300

Glu Glu Leu Lys Pro Leu Glu Glu Val Leu Asn Leu Ala Gln Ser Lys
305                 310                 315                 320

Asn Phe His Leu Arg Pro Arg Asp Leu Ile Ser Asn Ile Asn Val Ile
                325                 330                 335

Val Leu Glu Leu Lys Gly Ser Glu Thr Thr Phe Met Cys Glu Tyr Ala
            340                 345                 350

Asp Glu Thr Ala Thr Ile Val Glu Phe Leu Asn Arg Trp Ile Thr Phe
        355                 360                 365

Cys Gln Ser Ile Ile Ser Thr Leu Thr
    370                 375

<210>  18
<211>  377
<212>  PRT
<213>  Artificial Sequence

<220>
<223>  L19-IL2 (Diabody linker 3: GSADG)

<400>  18

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ala Asp Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Ser Ser Ser Ser Gly Ser Ser Ser Ser Gly Ser
225                 230                 235                 240

Ser Ser Ser Gly Ala Pro Thr Ser Ser Ser Thr Lys Lys Thr Gln Leu
                245                 250                 255

Gln Leu Glu His Leu Leu Leu Asp Leu Gln Met Ile Leu Asn Gly Ile
            260                 265                 270

Asn Asn Tyr Lys Asn Pro Lys Leu Thr Arg Met Leu Thr Phe Lys Phe
        275                 280                 285

Tyr Met Pro Lys Lys Ala Thr Glu Leu Lys His Leu Gln Cys Leu Glu
    290                 295                 300

Glu Glu Leu Lys Pro Leu Glu Glu Val Leu Asn Leu Ala Gln Ser Lys
305                 310                 315                 320

Asn Phe His Leu Arg Pro Arg Asp Leu Ile Ser Asn Ile Asn Val Ile
                325                 330                 335

Val Leu Glu Leu Lys Gly Ser Glu Thr Thr Phe Met Cys Glu Tyr Ala
            340                 345                 350

Asp Glu Thr Ala Thr Ile Val Glu Phe Leu Asn Arg Trp Ile Thr Phe
        355                 360                 365

Cys Gln Ser Ile Ile Ser Thr Leu Thr
    370                 375

<210>  19
<211>  377
<212>  PRT
<213>  Artificial Sequence

<220>
<223>  L19-IL2 (Diabody linker 4: GSAKG)

<400>  19

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Ser Ala Lys Gly Glu Ile Val Leu Thr Gln Ser
        115                 120                 125

Pro Gly Thr Leu Ser Leu Ser Pro Gly Glu Arg Ala Thr Leu Ser Cys
    130                 135                 140

Arg Ala Ser Gln Ser Val Ser Ser Ser Phe Leu Ala Trp Tyr Gln Gln
145                 150                 155                 160

Lys Pro Gly Gln Ala Pro Arg Leu Leu Ile Tyr Tyr Ala Ser Ser Arg
                165                 170                 175

Ala Thr Gly Ile Pro Asp Arg Phe Ser Gly Ser Gly Ser Gly Thr Asp
            180                 185                 190

Phe Thr Leu Thr Ile Ser Arg Leu Glu Pro Glu Asp Phe Ala Val Tyr
        195                 200                 205

Tyr Cys Gln Gln Thr Gly Arg Ile Pro Pro Thr Phe Gly Gln Gly Thr
    210                 215                 220

Lys Val Glu Ile Lys Ser Ser Ser Ser Gly Ser Ser Ser Ser Gly Ser
225                 230                 235                 240

Ser Ser Ser Gly Ala Pro Thr Ser Ser Ser Thr Lys Lys Thr Gln Leu
                245                 250                 255

Gln Leu Glu His Leu Leu Leu Asp Leu Gln Met Ile Leu Asn Gly Ile
            260                 265                 270

Asn Asn Tyr Lys Asn Pro Lys Leu Thr Arg Met Leu Thr Phe Lys Phe
        275                 280                 285

Tyr Met Pro Lys Lys Ala Thr Glu Leu Lys His Leu Gln Cys Leu Glu
    290                 295                 300

Glu Glu Leu Lys Pro Leu Glu Glu Val Leu Asn Leu Ala Gln Ser Lys
305                 310                 315                 320

Asn Phe His Leu Arg Pro Arg Asp Leu Ile Ser Asn Ile Asn Val Ile
                325                 330                 335

Val Leu Glu Leu Lys Gly Ser Glu Thr Thr Phe Met Cys Glu Tyr Ala
            340                 345                 350

Asp Glu Thr Ala Thr Ile Val Glu Phe Leu Asn Arg Trp Ile Thr Phe
        355                 360                 365

Cys Gln Ser Ile Ile Ser Thr Leu Thr
    370                 375

<210>  20
<211>  769
<212>  PRT
<213>  Artificial Sequence

<220>
<223>  L19-IL2 (scDb X 2)

<400>  20

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Cys Gln Ser Ile
        115                 120                 125

Ile Ser Thr Leu Thr Gly Gly Gly Gly Ser Gly Gly Gly Gly Ser Gly
    130                 135                 140

Gly Gly Gly Ser Glu Val Gln Leu Leu Glu Ser Gly Gly Gly Leu Val

145                 150                 155                 160

Gln Pro Gly Gly Ser Leu Arg Leu Ser Cys Ala Ala Ser Gly Phe Thr
                165                 170                 175

Phe Ser Ser Phe Ser Met Ser Trp Val Arg Gln Ala Pro Gly Lys Gly
            180                 185                 190

Leu Glu Trp Val Ser Ser Ile Ser Gly Ser Ser Gly Thr Thr Tyr Tyr
        195                 200                 205

Ala Asp Ser Val Lys Gly Arg Phe Thr Ile Ser Arg Asp Asn Ser Lys
    210                 215                 220

Asn Thr Leu Tyr Leu Gln Met Asn Ser Leu Arg Ala Glu Asp Thr Ala
225                 230                 235                 240

Val Tyr Tyr Cys Ala Lys Pro Phe Pro Tyr Phe Asp Tyr Trp Gly Gln
                245                 250                 255

Gly Thr Leu Val Thr Val Ser Ser Gly Ser Ser Gly Gly Glu Ile Val
            260                 265                 270

Leu Thr Gln Ser Pro Gly Thr Leu Ser Leu Ser Pro Gly Glu Arg Ala
        275                 280                 285

Thr Leu Ser Cys Arg Ala Ser Gln Ser Val Ser Ser Ser Phe Leu Ala
    290                 295                 300

Trp Tyr Gln Gln Lys Pro Gly Gln Ala Pro Arg Leu Leu Ile Tyr Tyr
305                 310                 315                 320

Ala Ser Ser Arg Ala Thr Gly Ile Pro Asp Arg Phe Ser Gly Ser Gly
                325                 330                 335

Ser Gly Thr Asp Phe Thr Leu Thr Ile Ser Arg Leu Glu Pro Glu Asp
            340                 345                 350

Phe Ala Val Tyr Tyr Cys Gln Gln Thr Gly Arg Ile Pro Pro Thr Phe
        355                 360                 365

Gly Gln Gly Thr Lys Val Glu Ile Lys Gly Ser Leu Asp Gly Ala Gly
    370                 375                 380

Gly Ser Ala Gly Ala Asp Gly Gly Glu Val Gln Leu Leu Glu Ser Gly
385                 390                 395                 400

Gly Gly Leu Val Gln Pro Gly Gly Ser Leu Arg Leu Ser Cys Ala Ala
                405                 410                 415

Ser Gly Phe Thr Phe Ser Ser Phe Ser Met Ser Trp Val Arg Gln Ala
            420                 425                 430

Pro Gly Lys Gly Leu Glu Trp Val Ser Ser Ile Ser Gly Ser Ser Gly
        435                 440                 445

Thr Thr Tyr Tyr Ala Asp Ser Val Lys Gly Arg Phe Thr Ile Ser Arg
    450                 455                 460

Asp Asn Ser Lys Asn Thr Leu Tyr Leu Gln Met Asn Ser Leu Arg Ala
465                 470                 475                 480

Glu Asp Thr Ala Val Tyr Tyr Cys Ala Lys Pro Phe Pro Tyr Phe Asp
                485                 490                 495

Tyr Trp Gly Gln Gly Thr Leu Val Thr Val Ser Ser Gly Ser Ser Gly
            500                 505                 510

Gly Glu Ile Val Leu Thr Gln Ser Pro Gly Thr Leu Ser Leu Ser Pro
        515                 520                 525

Gly Glu Arg Ala Thr Leu Ser Cys Arg Ala Ser Gln Ser Val Ser Ser
    530                 535                 540

Ser Phe Leu Ala Trp Tyr Gln Gln Lys Pro Gly Gln Ala Pro Arg Leu
545                 550                 555                 560

Leu Ile Tyr Tyr Ala Ser Ser Arg Ala Thr Gly Ile Pro Asp Arg Phe
                565                 570                 575

Ser Gly Ser Gly Ser Gly Thr Asp Phe Thr Leu Thr Ile Ser Arg Leu
            580                 585                 590

Glu Pro Glu Asp Phe Ala Val Tyr Tyr Cys Gln Gln Thr Gly Arg Ile
        595                 600                 605

Pro Pro Thr Phe Gly Gln Gly Thr Lys Val Glu Ile Lys Gly Gly Gly
    610                 615                 620

Gly Ser Gly Gly Gly Gly Ser Gly Gly Gly Gly Ser Ala Pro Thr Ser
625                 630                 635                 640

Ser Ser Thr Lys Lys Thr Gln Leu Gln Leu Glu His Leu Leu Leu Asp
                645                 650                 655

Leu Gln Met Ile Leu Asn Gly Ile Asn Asn Tyr Lys Asn Pro Lys Leu
            660                 665                 670

Thr Arg Met Leu Thr Phe Lys Phe Tyr Met Pro Lys Lys Ala Thr Glu
        675                 680                 685

Leu Lys His Leu Gln Cys Leu Glu Glu Glu Leu Lys Pro Leu Glu Glu
    690                 695                 700

Val Leu Asn Leu Ala Gln Ser Lys Asn Phe His Leu Arg Pro Arg Asp
705                 710                 715                 720

Leu Ile Ser Asn Ile Asn Val Ile Val Leu Glu Leu Lys Gly Ser Glu
                725                 730                 735

Thr Thr Phe Met Cys Glu Tyr Ala Asp Glu Thr Ala Thr Ile Val Glu
            740                 745                 750

Phe Leu Asn Arg Trp Ile Thr Phe Cys Gln Ser Ile Ile Ser Thr Leu
        755                 760                 765

Thr

<210>  21
<211>  5
<212>  PRT
<213>  Artificial Sequence

<220>
<223>  Linker between VH and VL in diabody and scDb

<400>  21

Gly Ser Ser Gly Gly
1               5

<210>  22
<211>  5
<212>  PRT
<213>  Artificial Sequence

<220>
<223>  Linker between VH and VL in diabody

<400>  22

Gly Gly Ser Gly Gly
1               5

<210>  23
<211>  5

<212>  PRT
<213>  Artificial Sequence

<220>
<223>  Linker between VH and VL in diabody

<400>  23

Gly Ser Ala Asp Gly
1               5


<210>  24
<211>  5
<212>  PRT
<213>  Artificial Sequence

<220>
<223>  Linker between VH and VL in diabody

<400>  24

Gly Ser Ala Lys Gly
1               5


<210>  25
<211>  15
<212>  PRT
<213>  Artificial Sequence

<220>
<223>  Linker between Diabody in scDb

<400>  25

Gly Ser Leu Asp Gly Ala Gly Gly Ser Ala Gly Ala Asp Gly Gly
1               5                   10                  15


<210>  26
<211>  15
<212>  PRT
<213>  Artificial Sequence

<220>
<223>  Linker between VL or VH and IL2

<400>  26

Gly Gly Gly Gly Ser Gly Gly Gly Gly Ser Gly Gly Gly Gly Ser
1               5                   10                  15


<210>  27
<211>  20
<212>  DNA
<213>  Artificial Sequence

<220>
<223>  Clones GGSGG, GSADG, and GSAKG Fragment A Forward Primer

<400>  27

taatacgact cactataggg                                                    20

<210>  28
<211>  37
<212>  DNA
<213>  Artificial Sequence

<220>
<223>  Clone GGSGG Fragment A Backward Primer

<400>  28
caccgcctga tcccccactc gagacggtga ccagggt                                 37

<210>  29
<211>  38
<212>  DNA
<213>  Artificial Sequence

<220>
<223>  Clone GSADG Fragment A Backward Primer

<400>  29
cgtctgctga cccactcgag acggtgacca gggttccc                                38

<210>  30
<211>  40
<212>  DNA
<213>  Artificial Sequence

<220>
<223>  Clone GSAKG Fragment A Backward Primer

<400>  30
accttttgct gacccactcg agacggtgac cagggttccc                               40

<210>  31
<211>  41
<212>  DNA
<213>  Artificial Sequence

<220>
<223>  Clone GGSGG Fragment B Forward Primer

<400>  31
tctcgagtgg gggatcaggc ggtgaaattg tgttgacgca g                            41

<210>  32
<211>  18
<212>  DNA
<213>  Artificial Sequence

<220>
<223>  Clone GGSGG, GSADG, and GSAKG Fragment B Backward Primer

<400>  32
tagaaggcac agtcgagg                                                      18

<210>  33
<211>  47
<212>  DNA
<213>  Artificial Sequence

<220>
<223>  Clone GSADG Fragment B Forward Primer

<400>  33
ccgtctcgag tgggtcagca gacggtgaaa ttgtgttgac gcagtct                47


<210>  34
<211>  47
<212>  DNA
<213>  Artificial Sequence

<220>
<223>  Clone GSAKG Fragment B Forward Primer

<400>  34
ccgtctcgag tgggtcagca aaaggtgaaa ttgtgttgac gcagtct                47


<210>  35
<211>  59
<212>  DNA
<213>  Artificial Sequence

<220>
<223>  Primer NheLead>

<400>  35
ctagctagct aggaccatgg gctggagcct gatcctcctg ttcctcgtcg ctgtggcta      59


<210>  36
<211>  59
<212>  DNA
<213>  Artificial Sequence

<220>
<223>  Primer IL2G4S3<

<400>  36
ccgccagaac cccctccgcc tgacccgcct ccaccagtca gtgttgagat gatgctttg      59


<210>  37
<211>  59
<212>  DNA
<213>  Artificial Sequence

<220>
<223>  Primer G4S3L19>

<400>  37
ggtcaggcgg agggggttct ggcggtggcg gatcggaggt gcagctgttg gagtctggg      59


<210>  38
<211>  33
<212>  DNA

<213>  Artificial Sequence

<220>
<223>  Primer L19Hind<

<400>  38
gaagcttcct ttgatttcca ccttggtccc ttg                                    33


<210>  39
<211>  59
<212>  DNA
<213>  Artificial Sequence

<220>
<223>  Primer LnkDP47>

<400>  39
atggagcagg tggcagtgca ggagcggacg ggggtgaggt gcagctgttg gagtctggg        59


<210>  40
<211>  33
<212>  DNA
<213>  Artificial Sequence

<220>
<223>  Primer HindLnk>

<400>  40
caagcttgga tggagcaggt ggcagtgcag gag                                    33


<210>  41
<211>  59
<212>  DNA
<213>  Artificial Sequence

<220>
<223>  Primer G4S3IL2>

<400>  41
ggtcaggcgg agggggttct ggcggtggcg gatcggcacc tacttcaagt tctacaaag        59


<210>  42
<211>  59
<212>  DNA
<213>  Artificial Sequence

<220>
<223>  Primer L19G4S3<

<400>  42
ccgccagaac cccctccgcc tgacccgcct ccacctttga tttccacctt ggtcccttg        59


<210>  43
<211>  57
<212>  DNA
<213>  Artificial Sequence

<220>

<223>  Primer IL2G4S3Bam<

<400>  43
cgcggatccc cctccgcctg acccgcctcc accagtcagt gttgagatga tgctttg          57


<210>  44
<211>  48
<212>  DNA
<213>  Artificial Sequence

<220>
<223>  Primer BamG4S3L19>

<400>  44
cgcggatccg gcggtggcgg atcggaggtg cagctgttgg agtctggg                    48


<210>  45
<211>  48
<212>  DNA
<213>  Artificial Sequence

<220>
<223>  Primer L19StopNot<

<400>  45
ttttcctttt gcggccgctc attatttgat ttccaccttg gtcccttg                    48


<210>  46
<211>  48
<212>  DNA
<213>  Artificial Sequence

<220>
<223>  Primer IL2StopNot<

<400>  46
ttttcctttt gcggccgctc attaagtcag tgttgagatg atgctttg                    48


<210>  47
<211>  57
<212>  DNA
<213>  Artificial Sequence

<220>
<223>  Primer DP47G4S2.5<

<400>  47
ccctgaccct ccgccaccag agcccccacc tccactcgag acggtgacca gggttcc          57


<210>  48
<211>  58
<212>  DNA
<213>  Artificial Sequence

<220>
<223>  Primer G4S2.5VL>

<400>  48

gggctctggt ggcggagggt cagggggagg cggtgaaatt gtgttgacgc agtctcca 58

<210> 49
<211> 59
<212> DNA
<213> Artificial Sequence

<220>
<223> Primer LeadIL2>

<400> 49
ctgttcctcg tcgctgtggc tacaggtgtg cactcggcac ctacttcaag ttctacaaa 59

<210> 50
<211> 29
<212> DNA
<213> Artificial Sequence

<220>
<223> Primer HindIIISIP

<400> 50
cccaagcttg tcgaccatgg gctggagcc 29

<210> 51
<211> 56
<212> DNA
<213> Artificial Sequence

<220>
<223> Primer L19Linker

<400> 51
gagccggaag agctactacc cgatgaggaa gatttgattt ccaccttggt cccttg 56

<210> 52
<211> 57
<212> DNA
<213> Artificial Sequence

<220>
<223> Primer LinkerIL2

<400> 52
tcgggtagta gctcttccgg ctcatcgtcc agcggcgcac ctacttcaag ttctaca 57

<210> 53
<211> 42
<212> DNA
<213> Artificial Sequence

<220>
<223> Primer IL2stopNotI

<400> 53
ttttcctttt gcggccgctc attaagtcag tgttgagatg at 42

<210>  54
<211>  118
<212>  PRT
<213>  Artificial Sequence

<220>
<223>  F8 VH

<400>  54

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Leu Phe
            20                  25                  30

Thr Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Thr His Leu Tyr Leu Phe Asp Tyr Trp Gly Gln Gly Thr
            100                 105                 110

Leu Val Thr Val Ser Ser
        115


<210>  55
<211>  108
<212>  PRT
<213>  Artificial Sequence

<220>
<223>  F8 VL

<400>  55

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Met Pro
            20                  25                  30

Phe Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Met Arg Gly Arg Pro
                85                  90                  95

Pro Thr Phe Gly Gln Gly Thr Lys Val Glu Ile Lys
            100                 105

<210>  56
<211>  3
<212>  PRT
<213>  Artificial Sequence

<220>
<223>  F8 VH CDR1

<400>  56

Leu Phe Thr
1

<210>  57
<211>  6
<212>  PRT
<213>  Artificial Sequence

<220>
<223>  F8 VH CDR2

<400>  57

Ser Gly Ser Gly Gly Ser
1               5

<210>  58
<211>  6
<212>  PRT
<213>  Artificial Sequence

<220>
<223>  F8 VH CDR3

<400>  58

Ser Thr His Leu Tyr Leu
1               5

<210>  59
<211>  3
<212>  PRT
<213>  Artificial Sequence

<220>
<223>  F8 VL CDR1

<400>  59

Met Pro Phe
1


<210>  60
<211>  7
<212>  PRT
<213>  Artificial Sequence

<220>
<223>  F8 VL CDR2

<400>  60

Gly Ala Ser Ser Arg Ala Thr
1               5


<210>  61
<211>  6
<212>  PRT
<213>  Artificial Sequence

<220>
<223>  F8 VL CDR3

<400>  61

Met Arg Gly Arg Pro Pro
1               5


<210>  62
<211>  477
<212>  PRT
<213>  Artificial Sequence

<220>
<223>  F8 scDb

<400>  62

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Leu Phe
            20                  25                  30


Thr Met Ser Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Thr His Leu Tyr Leu Phe Asp Tyr Trp Gly Gln Gly Thr
            100                 105                 110

Leu Val Thr Val Ser Ser Gly Ser Ser Gly Gly Glu Ile Val Leu Thr
        115                 120                 125

Gln Ser Pro Gly Thr Leu Ser Leu Ser Pro Gly Glu Arg Ala Thr Leu
    130                 135                 140

Ser Cys Arg Ala Ser Gln Ser Val Ser Met Pro Phe Leu Ala Trp Tyr
145                 150                 155                 160

Gln Gln Lys Pro Gly Gln Ala Pro Arg Leu Leu Ile Tyr Gly Ala Ser
                165                 170                 175

Ser Arg Ala Thr Gly Ile Pro Asp Arg Phe Ser Gly Ser Gly Ser Gly
            180                 185                 190

Thr Asp Phe Thr Leu Thr Ile Ser Arg Leu Glu Pro Glu Asp Phe Ala
        195                 200                 205

Val Tyr Tyr Cys Gln Gln Met Arg Gly Arg Pro Pro Thr Phe Gly Gln
    210                 215                 220

Gly Thr Lys Val Glu Ile Lys Gly Ser Leu Asp Gly Ala Gly Gly Ser
225                 230                 235                 240

Ala Gly Ala Asp Gly Gly Glu Val Gln Leu Leu Glu Ser Gly Gly Gly
                245                 250                 255

Leu Val Gln Pro Gly Gly Ser Leu Arg Leu Ser Cys Ala Ala Ser Gly
            260                 265                 270

Phe Thr Phe Ser Leu Phe Thr Met Ser Trp Val Arg Gln Ala Pro Gly
        275                 280                 285

Lys Gly Leu Glu Trp Val Ser Ala Ile Ser Gly Ser Gly Gly Ser Thr
    290                 295                 300

Tyr Tyr Ala Asp Ser Val Lys Gly Arg Phe Thr Ile Ser Arg Asp Asn
305                 310                 315                 320

Ser Lys Asn Thr Leu Tyr Leu Gln Met Asn Ser Leu Arg Ala Glu Asp
                325                 330                 335

Thr Ala Val Tyr Tyr Cys Ala Lys Ser Thr His Leu Tyr Leu Phe Asp
            340                 345                 350

Tyr Trp Gly Gln Gly Thr Leu Val Thr Val Ser Ser Gly Ser Ser Gly
        355                 360                 365

Gly Glu Ile Val Leu Thr Gln Ser Pro Gly Thr Leu Ser Leu Ser Pro
    370                 375                 380

Gly Glu Arg Ala Thr Leu Ser Cys Arg Ala Ser Gln Ser Val Ser Met
385                 390                 395                 400

Pro Phe Leu Ala Trp Tyr Gln Gln Lys Pro Gly Gln Ala Pro Arg Leu
                405                 410                 415

Leu Ile Tyr Gly Ala Ser Ser Arg Ala Thr Gly Ile Pro Asp Arg Phe
            420                 425                 430

Ser Gly Ser Gly Ser Gly Thr Asp Phe Thr Leu Thr Ile Ser Arg Leu
        435                 440                 445

Glu Pro Glu Asp Phe Ala Val Tyr Tyr Cys Gln Gln Met Arg Gly Arg
    450                 455                 460

Pro Pro Thr Phe Gly Gln Gly Thr Lys Val Glu Ile Lys
465                 470                 475

<210>  63
<211>  116
<212>  PRT
<213>  Artificial Sequence

<220>
<223>  F16 VH

<400>  63

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Gly Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala His Asn Ala Phe Asp Tyr Trp Gly Gln Gly Thr Leu Val
            100                 105                 110

Thr Val Ser Arg
        115

<210>  64
<211>  109
<212>  PRT
<213>  Artificial Sequence

<220>
<223>  F16 VL

<400>  64

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Asn Ser Ser Val Tyr Thr Met Pro Pro
                85                  90                  95

Val Val Phe Gly Gly Gly Thr Lys Leu Thr Val Leu Gly
            100                 105

<210>  65
<211>  5
<212>  PRT
<213>  Artificial Sequence

<220>
<223>  F16 VH CDR1

<400>  65

Arg Tyr Gly Met Ser
1               5


<210>  66
<211>  17
<212>  PRT
<213>  Artificial Sequence

<220>
<223>  F16 VH CDR2

<400>  66

Ala Ile Ser Gly Ser Gly Gly Ser Thr Tyr Tyr Ala Asp Ser Val Lys
1               5                   10                  15


Gly


<210>  67
<211>  7
<212>  PRT
<213>  Artificial Sequence

<220>
<223>  F16 VH CDR3

<400>  67

Ala His Asn Ala Phe Asp Tyr
1               5


<210>  68
<211>  11
<212>  PRT
<213>  Artificial Sequence

<220>
<223>  F16 VL CDR1

<400>  68

Gln Gly Asp Ser Leu Arg Ser Tyr Tyr Ala Ser
1               5                   10


<210>  69
<211>  7
<212>  PRT
<213>  Artificial Sequence

<220>
<223>  F16 VL CDR2

<400>  69

Gly Lys Asn Asn Arg Pro Ser
1               5

<210>  70
<211>  11
<212>  PRT
<213>  Artificial Sequence

<220>
<223>  F16 VL CDR3

<400>  70

Asn Ser Ser Val Tyr Thr Met Pro Pro Val Val
1               5                   10

<210>  71
<211>  475
<212>  PRT
<213>  Artificial Sequence

<220>
<223>  F16 scDb

<400>  71

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Gly Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala His Asn Ala Phe Asp Tyr Trp Gly Gln Gly Thr Leu Val
            100                 105                 110

Thr Val Ser Arg Gly Ser Ser Gly Gly Ser Ser Glu Leu Thr Gln Asp
        115                 120                 125

Pro Ala Val Ser Val Ala Leu Gly Gln Thr Val Arg Ile Thr Cys Gln
    130                 135                 140

Gly Asp Ser Leu Arg Ser Tyr Tyr Ala Ser Trp Tyr Gln Gln Lys Pro
145                 150                 155                 160

Gly Gln Ala Pro Val Leu Val Ile Tyr Gly Lys Asn Asn Arg Pro Ser
                165                 170                 175

Gly Ile Pro Asp Arg Phe Ser Gly Ser Ser Ser Gly Asn Thr Ala Ser
            180                 185                 190

Leu Thr Ile Thr Gly Ala Gln Ala Glu Asp Glu Ala Asp Tyr Tyr Cys
        195                 200                 205

Asn Ser Ser Val Tyr Thr Met Pro Pro Val Val Phe Gly Gly Gly Thr
    210                 215                 220

Lys Leu Thr Val Leu Gly Gly Ser Leu Asp Gly Ala Gly Gly Ser Ala
225                 230                 235                 240

Gly Ala Asp Gly Gly Glu Val Gln Leu Leu Glu Ser Gly Gly Gly Leu
                245                 250                 255

Val Gln Pro Gly Gly Ser Leu Arg Leu Ser Cys Ala Ala Ser Gly Phe
            260                 265                 270

Thr Phe Ser Arg Tyr Gly Met Ser Trp Val Arg Gln Ala Pro Gly Lys
        275                 280                 285

Gly Leu Glu Trp Val Ser Ala Ile Ser Gly Ser Gly Gly Ser Thr Tyr
    290                 295                 300

Tyr Ala Asp Ser Val Lys Gly Arg Phe Thr Ile Ser Arg Asp Asn Ser
305                 310                 315                 320

Lys Asn Thr Leu Tyr Leu Gln Met Asn Ser Leu Arg Ala Glu Asp Thr
                325                 330                 335

Ala Val Tyr Tyr Cys Ala Lys Ala His Asn Ala Phe Asp Tyr Trp Gly
            340                 345                 350

Gln Gly Thr Leu Val Thr Val Ser Arg Gly Ser Ser Gly Gly Ser Ser
        355                 360                 365

Glu Leu Thr Gln Asp Pro Ala Val Ser Val Ala Leu Gly Gln Thr Val
    370                 375                 380

Arg Ile Thr Cys Gln Gly Asp Ser Leu Arg Ser Tyr Tyr Ala Ser Trp
385                 390                 395                 400

Tyr Gln Gln Lys Pro Gly Gln Ala Pro Val Leu Val Ile Tyr Gly Lys
                405                 410                 415

Asn Asn Arg Pro Ser Gly Ile Pro Asp Arg Phe Ser Gly Ser Ser Ser
            420                 425                 430

Gly Asn Thr Ala Ser Leu Thr Ile Thr Gly Ala Gln Ala Glu Asp Glu
        435                 440                 445

Ala Asp Tyr Tyr Cys Asn Ser Ser Val Tyr Thr Met Pro Pro Val Val
    450                 455                 460

Phe Gly Gly Gly Thr Lys Leu Thr Val Leu Gly
465                 470                 475